# EUROPEAN PATENT APPLICATION

(11) **EP 3 841 990 A1**
(43) Date of publication of application: **30.06.2021**
(21) Application number: 19852592.5
(22) Date of filing: 23.08.2019
(51) Int. Cl.: A61B 17/29, A61B 17/062

(54) **GRASPER, NEEDLE CARRIER, AND PINCHING ATTACHMENT**

(30) Priority: 24.08.2018 JP 2018157545
(71) Applicant: National Industry Information Research Institute Co., Ltd., Tokyo, 140-0015 (JP)
(72) Inventor: MOTOMURA Noboru, Sakura-shi, Chiba 285-8741 (JP); NAKAYA Mitsuhiro, Tokyo 140-0015 (JP); YUBA Hirokazu, Ikeda-shi, Osaka 563-0035 (JP)
(74) Representative: Plasseraud IP
(86) International application number: PCT/JP2019/033058
(87) International publication number: WO 2020/040295

(57) **Abstract**

A needle carrier (100) includes a pinching attachment (20) having a pinching arm member and a pinching arm support portion on which the pinching arm member is disposed and configured to pivotably support the pinching arm member around a first axis line, a needle carrier body (10) on which a grip portion (112) is formed, a pinching arm opening/closing drive portion (30) having a pinching operation lever (31) and a pinching operation transmission part, and a pinching arm pivotal drive portion (40) having a pivotal operation wheel (41) and a pivotal operation transmission part, and the pinching arm opening/closing drive portion (30) includes an axial dimension expansion/contraction spring (35) configured to attenuate a pinching operating force input from the pinching operation lever (31) and pinch the pinching portions.

## Description

### [Technical Field]

The present invention relates to a grasper configured to allow handling of a surgical instrument, biological tissue, or the like, in a vertical tangential direction in a confined space or the like, a needle carrier configured to enable handling of a needle in the vertical tangential direction, and a pinching attachment used for the grasper and the needle carrier.

Priority is claimed on Japanese Patent Application No. 2018-157545, filed August 24, 2018, the content of which is incorporated herein by reference.

### [Background Art]

As is well known, under various environmental conditions in surgical operations, techniques such as grasping, piercing, suturing, or the like, are performed on a living body. For this reason, various graspers and needle carriers including forceps are used (for example, see Patent Literature 1).

In the related art, when biological tissue or the like is sutured, in general, a suturing needle is grasped in a lateral direction perpendicular to a longitudinal direction of a needle carrier, and the needle carrier is handled by being twisted around in the longitudinal direction.

In recent years, minimally invasive surgery in a confined space using an operation of an endoscope has become more common, and since in minimally invasive surgery in a confined space, a suturing needle or the like can be efficiently pinched in a narrow field of view, the necessity for efficient handling is increasing.

Here, in order to efficiently perform a grasping operation or a suturing operation in a confined space, for example, a grasper or a needle carrier in which a suturing needle can be handled in a longitudinal direction of the needle carrier (hereinafter, may be referred to as a vertical tangential direction) has been developed (for example, see Patent Literature 2).

Since the grasper or the needle carrier disclosed in Patent Literature 2 handles an object such as a suturing needle or the like in a vertical tangential direction, this is better in that it is possible to proceed with surgery efficiently in a confined space.

However, since an arm portion of a forceps main body needs to be opened and closed when an object is pinched in the grasper and the needle carrier disclosed in Patent Literature 2, it may not always be easy to perform a pinching operation in a deep surgical field at a position distant from outside of the body or a pinching operation through a trocar having a small diameter.

Here, a grasper and a needle carrier that can easily insert a pinching portion into a surgical field via a trocar and efficiently perform a pinching operation or a pivotal operation in a confined space by providing a configuration in which members from a pinching operation input part and a pivotal operation input part to the pinching portion can be housed in a pipe has been disclosed (for example, see Patent Literature 3).

This is better in that the grasper and the needle carrier disclosed in Patent Literature 3 can easily and efficiently pinch an object such as a suturing needle or the like in a confined space, and moreover, the object can be efficiently handled in the vertical tangential direction.

### [Citation List]

### [Patent Literature]

[Patent Literature 1]
   Japanese Unexamined Patent Application, First Publication No. H08-038492
[Patent Literature 2]
   Japanese Unexamined Patent Application, First Publication No. 2013-111119
[Patent Literature 3]
   Japanese Unexamined Patent Application, First Publication No. 2018-000391

### [Summary of Invention]

### [Technical Problem]

However, in the grasper disclosed in Patent Literature 3, since the closed end of the pinching portion when an object is pinched is configured to be uniformly determined according to a position of a ratchet, the closed end is determined regardless of the strength or the like of the object when a pinching operation is performed. In addition, since the force is directly transmitted to the object when the object is pinched, if a pinching operating force is too large, deformation, damage, or the like, may occur in the object. Meanwhile, the ratchet may be engaged before the object is sufficiently pinched when the pinching operating force is small, and the pinching portion may not sufficiently pinch the object, which may cause misalignment, dropping out, or the like.

In addition, since it is desirable to use the pinching member as a disposable item, the pinching member should be easily and efficiently mounted on the grasper and the needle carrier, and it is desirable to use a pinching attachment (the detachable pinching member) that can be reliably held by the grasper or the like.

In consideration of the above-mentioned circumstances, the present invention is directed to realizing at least one of the following:
(1) Providing a grasper and a needle carrier that can be easily and efficiently operated by improving the reliability of a pinching operation when an object is pinched, and
(2) Providing a pinching attachment that can be easily and efficiently mounted on a grasper main body and reliably held on the grasper or the like.

### [Solution to Problem]

In order to solve the above-mentioned problems, the present invention provides the following means.
(1) A first aspect of the present invention is a grasper configured to grasp an object, including: a grasping member including a pair of pinching arm members having pinching portions formed on a tip side in a longitudinal direction and configured to pinch an object, which relatively pivot around a first axis line to cause the pinching portions to approach each other or separate from each other, and a pinching arm holding portion on which the pair of pinching arm members are pivotably disposed around the first axis line; a first drive part including a pinching operation part configured to pinch the pair of pinching arm members and a pinching operation transmission part configured to transmit a pinching operation from the pinching operation part to the pair of pinching arm members, in which the first drive part is configured to cause the pinching portions to approach each other according to the pinching operation input to the pinching operation part; a second drive part including a pivotal operation part configured to pivot the pair of pinching arm members, and a grasping member pivoting portion configured to pivot the grasping member around a longitudinal direction of the pinching arm member and around a second axis line perpendicular to the first axis line according to the pivotal operation input from the pivotal operation part; and an elongated grasper main body having a grip portion held by an operator in an orientation crossing the longitudinal direction, in which the grasping member, the first drive part and the second drive part are disposed on the elongated grasper main body, wherein the first drive part includes a pinching operation displacement absorption means configured to bias the pair of arm members to cause the pinching portions to relatively approach each other according to the pinching operation input from the pinching operation part.
   According to the aspect of the first aspect, since the grasping member including the pair of pinching arm members having the pinching portions formed on the tip side in the longitudinal direction and configured to pinch the object, which relatively pivot around the first axis line to cause the pinching portions to approach each other or be separated from each other, and the pinching arm holding portion on which the pair of pinching arm members are pivotably disposed around the first axis line; the first drive part including the pinching operation part configured to pinch the pair of pinching arm members, and the pinching operation transmission part configured to transmit the pinching operation from the pinching operation part to the pair of pinching arm members, in which the first drive part is configured to cause the pinching portions to approach each other according to the pinching operation input to the pinching operation part; the second drive part including the pivotal operation part configured to pivot the pair of pinching arm members, and the grasping member pivoting portion configured to pivot the grasping member around the longitudinal direction of the pinching arm member and around the second axis line perpendicular to the first axis line according to the pivotal operation input from the pivotal operation part; and the elongated grasper main body having the grip portion held by the operator in the orientation crossing the longitudinal direction, in which the grasping member, the first drive part and the second drive part are disposed on the elongated grasper main body, are provided, the operator can pinch the first drive part to cause the pinching portions to easily and efficiently approach each other (close) to pinch the object, and pivot the second drive part and pivot the pair of pinching arms to change a posture of the object (or vertically handle the needle).
   In addition, since the first drive part includes the pinching operation displacement absorption means and biases the pair of arm members to a side where the pinching portions relatively approach each other according to the pinching operation input from the pinching operation part to absorb displacement, occurrence of an insufficient pinching force or play when the pinching portions pinch the object can be minimized, and the object can be pinched by an appropriate pinching force.
   In addition, since the first drive part includes the pinching operation displacement absorption means and the displacement input by the pinching operation is absorbed by an elasticity applying means and not transmitted to the pinching portion as it is, even when a large force is input from the pinching operation part, occurrence of damage to the object and the member constituting the first drive part can be minimized.
   As a result, the reliability of the pinching operation when the object is pinched can be improved, and the operator can easily and efficiently operate the grasper.
   Here, causing the pinching portions to relatively approach each other or separate means causing the facing pinching portions (or pinching surfaces) to approach each other or separate while maintaining a posture in which they are parallel to each other, and making the pinching portions face each other in a posture appropriate for grasping an object in a state in which the pinching arm members are pivoted such that they are opened or closed and the pinching portions are made to approach each other or separate while changing orientations thereof.
   In addition, when the pinching portions relatively approach or are separated from each other, the pinching portions formed on the pair of pinching arm members may be moved together to approach each other, or one pinching portion may move and approach the other pinching portion.
   In addition, the longitudinal direction of the pinching arm member is referred to as a direction in which the pinching portions extend in a state in which the pinching arm members are closed.
   In addition, the pinching operation displacement absorption means is referred to as a member that enables absorption (attenuation) and outputting (transmission) of displacement input to the pinching operation when the pinching operation is input from the pinching operation part, and various configurations of absorbing and outputting the input displacement, for example, a member constituted by an elastic member such as an S-shaped spring, a coil spring, a spiral spring, a torsion spring, or the like, a member that absorbs displacement by compressing a fluid such as an air spring or the like constituted by an air cylinder closed at the outside, or the like, may be applied.
   In addition, the object includes a living body (including a part), a tissue, a piercing instrument, an incision instrument, a suturing needle, or the like.
(2) In the grasper according to the above-mentioned (1), the pinching operation displacement absorption means may be constituted by a flexible transmission member disposed on the pinching operation transmission part and operated by a pinching operation transmission force.
   According to the grasper of the above-mentioned (2), since the pinching operation displacement absorption means is constituted by the flexible transmission member disposed on the pinching operation transmission part and operated by the pinching operation transmission force, a biasing force (an elastic force) can be reliably applied to the pinching operation transmission with a simple configuration.
   In addition, when the pinching arm members are closed, it is possible to absorb the displacement due to the pinching operation and minimize an effect applied to the pinching portions, due to the flexible transmission member being deformed.
   Here, the flexible transmission member is referred to as a member whose pinching force associated with the pinching operation can be converted into deformation or displacement by an elastic force.
(3) In the grasper according to the above-mentioned (2), the pinching operation displacement absorption means may be constituted by an axial dimension expansion/contraction spring.
   According to the grasper of the above-mentioned (3), since the pinching operation displacement absorption means is constituted by the axial dimension expansion/contraction spring, the displacement due to the pinching operation can be absorbed by deforming the axial dimension expansion/contraction spring to prevent an excessive force from being applied to the pinching portions. In addition, the object can be reliably pinched.
(4) In the grasper according to the above-mentioned (2), the pinching operation displacement absorption means may be constituted by an axial dimension expansion/contraction spring having a circumferential portion surrounded by an elastic material.
   According to the grasper of the above-mentioned (4), since the pinching operation displacement absorption means is constituted by the axial dimension expansion/contraction spring having the circumferential portion surrounded by the elastic material, it is possible to prevent the excessive pinching force associated with the pinching operation from being applied to the pinching portions. In addition, the object can be reliably pinched.
(5) In the grasper according to the above-mentioned (2), the pinching operation displacement absorption means may be constituted by an S-shaped axial dimension expansion/contraction spring constituted by curved portions on which elastic materials are directed toward opposite sides.
   According to the grasper of the above-mentioned (5), since the pinching operation displacement absorption means is constituted by the S-shaped axial dimension expansion/contraction spring constituted by the curved portions on which the elastic materials are directed toward opposite sides, it is possible to prevent the excessive pinching force associated with the pinching operation from being applied to the pinching portion. In addition, the object can be reliably pinched.
(6) The grasper according to the above-mentioned (2) is the grasper according to any one of the above-mentioned (1) to (5), which may include a pinching operation lock mechanism configured to automatically hold a pinching state of the pinching portions by operating the pinching operation part.
   According to the grasper of the above-mentioned (6), since the pinching operation lock mechanism configured to automatically hold the pinching state of the pinching portions by operating the pinching operation part is provided, the pinching state of the pinching portion can be efficiently performed as a part of the pinching operation. In addition, the object can be pinched by a desired pinching force.
(7) The grasper according to any one of the above-mentioned (1) to (6) may include a pinching operation release mechanism configured to release the pinching state of the pinching portions using the pinching operation part by operating the pinching operation part in a state in which the pinching operation lock mechanism holds the pinching state of the pinching portions.
   According to the grasper of the above-mentioned (7), since the pinching operation release mechanism configured to release the pinching state of the pinching portions using the pinching operation part in a state in which the pinching operation lock mechanism holds the pinching state of the pinching portions is provided, the pinching state of the pinching portion can be efficiently released.
(8) A second aspect of the present invention is a needle carrier including the grasper according to any one of the above-mentioned (1) to (7), and a suturing needle pinching concave portion corresponding to a suturing needle may be formed in the pinching portions.
   According to the needle carrier of the above-mentioned (8), since the suturing needle pinching concave portion corresponding to the suturing needle is formed in the pinching portions, the suturing needle can be easily and stably grasped.
   As a result, the suturing needle can be stably grasped and efficiently and stably handled in a confined space or the like.
(9) A third aspect of the present invention is a pinching attachment including the first drive part according to any one of the above-mentioned (1) to (8) and configured to transmit a pinching operation to the pair of pinching arm members, and a grasping member pivoting portion constituting the second drive part and configured to pivot the grasping member around the second axis line, and used by being mounted on a grasper having a first engaging portion configured to detachably mount the grasping member on the grasping member pivoting portion, one of the pair of pinching arm members being connected to a tip side of the pinching arm holding portion and a pinching operation receiving lever configured to receive a pinching operation from the pinching arm operating lever being connected to a base end side of the other of the pair of pinching arm members, the pinching operation receiving lever being disposed at a position corresponding to the pinching arm operating lever via a through-hole formed from the tip side toward the base end side of the pinching arm holding portion, a second engaging portion corresponding to the first engaging portion being formed in the pinching arm holding portion on the base end side, the first engaging portion and the second engaging portion having flange portions configured to set an attachment position set in the attachment/detachment direction in a state in which a boss formed on any one thereof is inserted into a hole formed in the other and to prevent the boss from falling out in the attachment/detachment direction, and the flange portions being engaged with each other by being pivoted around an axis line in the attachment/detachment direction at the attachment position and causing the flange portions to overlap each other, and the pinching operation receiving lever being configured to be insertable between the pinching arm operating lever and the pinching arm holding portion by causing the pinching portions of the pair of pinching arm members to approach each other in a state in which the first engaging portion and the second engaging portion are twisted to a position in which they are movable in the attachment/detachment direction.

According to the pinching attachment of the above-mentioned (9), the first engaging portion and the second engaging portion are twisted to a position where the flange portions do not interfere with each other when they are moved in the attachment/detachment direction, and the boss formed in one of the first engaging portion and the second engaging portion is inserted into the hole formed in the other to be disposed at the attachment position in the attachment/detachment direction. Then, the first engaging portion and the second engaging portion are twisted around the axis line in the attachment/detachment direction and the flange portions thereof overlap each other, and the pinching attachment is mounted such that the first engaging portion and the second engaging portion do not fall out in the attachment/detachment direction.

In addition, when the boss is inserted into the hole, since the pinching portions of the pair of pinching arm members approach each other, the pinching operation receiving lever can be inserted between the pinching arm operating lever and the pinching arm holding portion.

As a result, the pinching attachment can be easily and efficiently mounted on the grasper main body.

### [Advantageous Effects of Invention]

According to a grasper and a needle carrier of the present invention, the grasper and the needle carrier can be easily and efficiently operated by improving the reliability of a pinching operation when an object is pinched.

In addition, according to a pinching attachment according to the present invention, the pinching attachment can be easily and efficiently mounted on a grasper main body and reliably held on the grasper or the like.

### [Brief Description of Drawings]

Fig. 1 is a longitudinal cross-sectional view explaining a schematic configuration of a needle carrier according to a first embodiment of the present invention when seen in a side view.
Fig. 2 is a perspective view explaining a schematic configuration in a state in which a pinching attachment according to the first embodiment is attached.
Fig. 3A is a plan view explaining a schematic configuration in a state in which the pinching attachment according to the first embodiment is open.
Fig. 3B is a plan view explaining a schematic configuration in a state in which the pinching attachment according to the first embodiment is closed.
Fig. 4 is a perspective view of the pinching attachment, which is a view explaining a schematic configuration of an attachment relationship between the pinching attachment and a grasping member pivoting portion according to the first embodiment.
Fig. 5A is a perspective view of the grasping member pivoting portion, which is a view explaining a schematic configuration of an attachment relationship between the pinching attachment and the grasping member pivoting portion according to the first embodiment.
Fig. 5B is a cross-sectional view taken along arrow VB-VB in Fig. 5A when the grasping member pivoting portion is seen from a base end side in an attachment/detachment direction, which is a view explaining a schematic configuration of the attachment relationship between the pinching attachment and the grasping member pivoting portion according to the first embodiment.
Fig. 6A is a perspective view showing a state in which the pinching attachment is twisted and inserted into the grasping member pivoting portion, which is a view explaining a schematic configuration of a mounting procedure of the pinching attachment according to the first embodiment.
Fig. 6B is a perspective view showing a state in which the pinching attachment is mounted on the grasping member pivoting portion, which is a view explaining a schematic configuration of a mounting procedure of the pinching attachment according to the first embodiment.
Fig. 6C is a perspective view showing a state in which a pair of pinching arm members of the pinching attachment engage a pinching operation receiving lever with a pinching arm operating lever, which is a view explaining a schematic configuration of a mounting procedure of the pinching attachment according to the first embodiment.
Fig. 7A is a cross-sectional view taken along arrow VB-VB in Fig. 5A when a state in which the pinching attachment is twisted with respect to the grasping member pivoting portion is seen from a base end side in the attachment/detachment direction, which is a view explaining a schematic configuration of an attachment relationship between the pinching attachment and the grasping member pivoting portion according to the first embodiment.
Fig. 7B is a cross-sectional view taken along arrow VB-VB in Fig. 5A when a state in which the pinching attachment is mounted on the grasping member pivoting portion is seen from the base end side in the attachment/detachment direction, which is a view explaining a schematic configuration of an attachment relationship between the pinching attachment and the grasping member pivoting portion according to the first embodiment.
Fig. 8 is a longitudinal cross-sectional view explaining a schematic configuration of a pinching arm opening/closing drive portion according to the first embodiment when seen in a side view.
Fig. 9 is a side view explaining major portions of the pinching arm opening/closing drive portion and the pinching arm pivotal drive portion according to the first embodiment in detail.
Fig. 10 is a perspective view explaining major portions of the pinching arm opening/closing drive portion and the pinching arm pivotal drive portion according to the first embodiment in detail.
Fig. 11 is a schematic configuration view showing a schematic configuration of a pinching operation lock mechanism of the pinching arm opening/closing drive portion according to the first embodiment when seen from a side view.
Fig. 12A is a schematic configuration view showing a state before a pinching operation is started, which is a view explaining an action of the pinching arm opening/closing drive portion and the pinching operation lock mechanism according to the first embodiment.
Fig. 12B is a schematic configuration view showing a state when the pinching operation is terminated, which is a view explaining an action of the pinching arm opening/closing drive portion and the pinching operation lock mechanism according to the first embodiment.
Fig. 13A is a schematic configuration view showing an axial dimension expansion/contraction spring in a state in which a pinching arm member is open, which is a view explaining an action of the pinching arm opening/closing drive portion according to the first embodiment.
Fig. 13B is a schematic configuration view showing the axial dimension expansion/contraction spring in a state in which the pinching arm member is closed, which is a view explaining an action of the pinching arm opening/closing drive portion according to the first embodiment.
Fig. 14A is a schematic configuration view showing a state in which release of the pinching operation lock mechanism is started, which is a view explaining release of the pinching operation lock mechanism according to the first embodiment.
Fig. 14B is a schematic configuration view showing a state in which the pinching operation lock mechanism is released, which is a view explaining release of the pinching operation lock mechanism according to the first embodiment.
Fig. 15 is a longitudinal cross-sectional view explaining a schematic configuration of the pinching arm pivotal drive portion according to the first embodiment when seen in a side view.
Fig. 16 is a schematic configuration view explaining an action of the pinching arm pivotal drive portion according to the first embodiment when seen in a side view.
Fig. 17A is a perspective view showing a state in which the suturing needle is pinched by the pinching arm member, which is a view explaining an example of an action of the needle carrier according to the first embodiment.
Fig. 17B is a conceptual view showing a state in which the suturing needle is pierced into a first biological tissue, which is a view explaining an example of an action of the needle carrier according to the first embodiment.
Fig. 17C is a conceptual view showing a state in which the suturing needle sutures a first biological tissue and a second biological tissue, which is a view explaining an example of an action of the needle carrier according to the first embodiment.
Fig. 17D is a conceptual view showing a state in which the suturing needle that has sutured the first biological tissue and the second biological tissue is exchanged, which is a view explaining an example of an action of the needle carrier according to the first embodiment.
Fig. 18 is a longitudinal cross-sectional view explaining a schematic configuration of a needle carrier according to a second embodiment of the present invention when seen in a side view.
Fig. 19 is a longitudinal cross-sectional view explaining a schematic configuration of a pinching arm pivotal drive portion according to the second embodiment when seen in a side view.
Fig. 20 is a longitudinal cross-sectional view explaining a schematic configuration of a pinching arm opening/closing drive portion according to the second embodiment when seen in a side view.
Fig. 21 is a side view explaining major portions of the pinching arm opening/closing drive portion and the pinching arm pivotal drive portion according to the second embodiment in detail.
Fig. 22A is a schematic configuration view showing an S-shaped spring in a state in which a pinching arm member is open, which is a view explaining an action of the pinching arm opening/closing drive portion according to the second embodiment.
Fig. 22B is a schematic configuration view showing the S-shaped spring in a state in which the pinching arm member is closed, which is a view explaining an action of the pinching arm opening/closing drive portion according to the second embodiment.
Fig. 23 is a longitudinal cross-sectional view explaining a schematic configuration of a grasper according to a third embodiment of the present invention when seen in a side view.
Fig. 24A is a schematic configuration view explaining a schematic configuration of a forceps attachment according to the third embodiment of the present invention when seen in a side view.
Fig. 24B is a schematic configuration view explaining a schematic configuration of the forceps attachment according to the third embodiment of the present invention when seen in a plan view.
Fig. 24C is a schematic configuration view explaining a schematic configuration of the forceps attachment according to the third embodiment of the present invention when seen from a base end side.
Fig. 25A is a conceptual view showing a first posture when biological tissue is treated while a posture thereof is changed in the abdominal cavity, which is a view explaining an example of an action of the grasper according to the third embodiment of the present invention.
Fig. 25B is a conceptual view showing a second posture when biological tissue is treated while a posture thereof is changed in the abdominal cavity, which is a view explaining an example of an action of the grasper according to the third embodiment.
Fig. 26A is a schematic configuration view explaining a schematic configuration of a forceps attachment according to a fourth embodiment of the present invention when seen in a side view.
Fig. 26B is a schematic configuration view explaining a schematic configuration of the forceps attachment according to the fourth embodiment of the present invention when seen in a plan view.
Fig. 26C is a schematic configuration view explaining a schematic configuration of the forceps attachment according to the fourth embodiment of the present invention when seen from a base end side.

### [Description of Embodiments]

### <First embodiment>

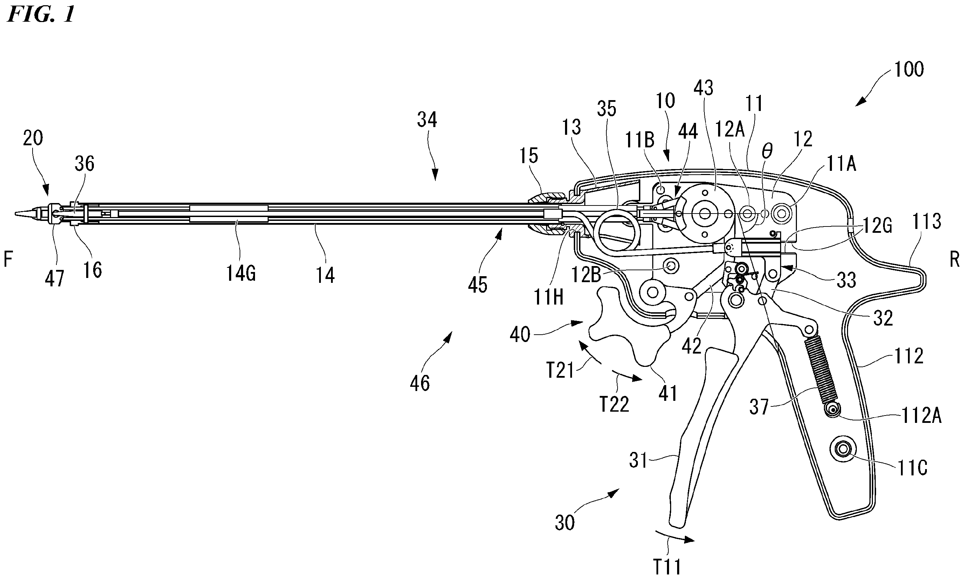

Hereinafter, a first embodiment of the present invention will be described with reference to Fig. 1 to Fig. 17D.

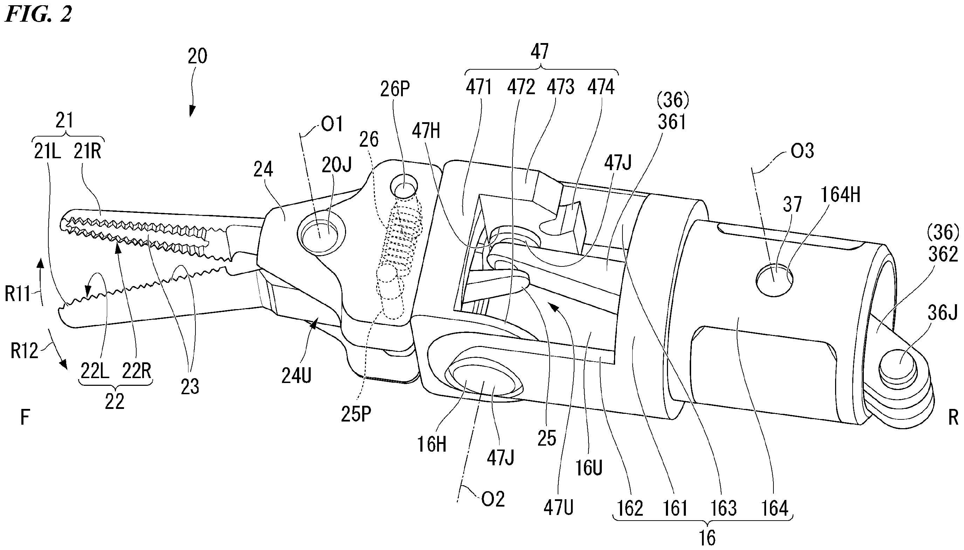

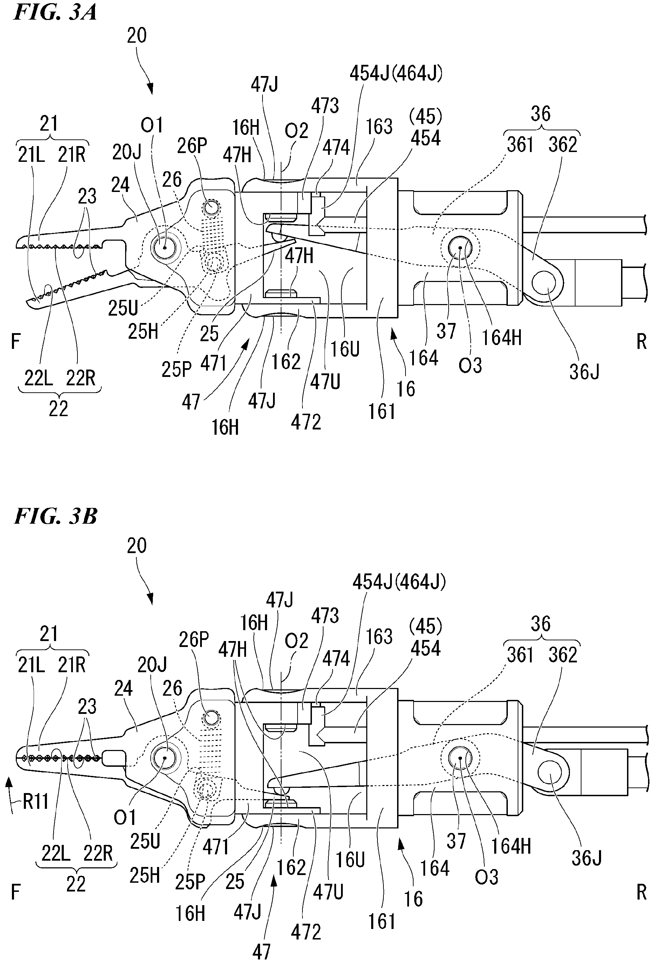

First, a schematic configuration of a needle carrier according to the first embodiment will be described with reference to Figs. 1 to 16. Fig. 1 is a longitudinal cross-sectional view explaining a schematic configuration of a needle carrier according to the first embodiment when seen from a side view, and Fig. 2 is a perspective view explaining a schematic configuration in a state in which a pinching attachment is attached. In addition, Fig. 3A is a plan view explaining a schematic configuration in a state in which the pinching attachment is open, and Fig. 3B is a plan view explaining a schematic configuration in a state in which the pinching attachment is closed.

In Figs. 1, 2, 3A and 3B, reference sign 100 designates a needle carrier (a grasper), reference sign 10 designates a needle carrier body (a grasper main body), reference sign 20 designates a pinching attachment (a grasping member), reference sign 30 designates a pinching arm opening/closing drive portion (a first drive part), and reference sign 40 designates a pinching arm pivotal drive portion (a second drive part). In addition, reference sign 21 designates a pair of pinching arm members (hereinafter, may be referred to as a pinching arm member), reference sign 22 designates a pinching portion, reference sign 24 designates a pinching arm holding portion, and reference sign 20J designates a pivoting support shaft. In addition, reference sign O1 designates a first axis line about which the pinching arm member 21 is pivoted to be opened and closed, reference sign O2 designates a second axis line about which the pinching arm member 21 is pivoted in a vertical tangential direction, reference sign F designates a tip side, and reference sign R designates a base end side (a side of an operator).

As shown in Fig. 1, the needle carrier (the grasper) 100 includes the needle carrier body (the grasper main body) 10, the pinching attachment (the grasping member) 20, the pinching arm opening/closing drive portion (the first drive part) 30, and the pinching arm pivotal drive portion (the second drive part) 40.

Furthermore, the needle carrier (the grasper) 100 is configured to handle a suturing needle (an object) in a surface in a vertical tangential direction (a longitudinal direction of the needle carrier 100) by pivoting the pinching attachment 20 around the second axis line O2 while pinching the suturing needle (the object) by pivoting the pinching arm member 21 around the first axis line O1.

Further, the needle carrier 100 may be used as a grasper configured to grasp biological tissue (including a part), a piercing instrument, an incision instrument, or the like, as an object, instead of the suturing needle.

For example, the needle carrier body (the grasper main body) 10 includes a casing 11, a base plate 12, a pipe attachment member 13, a protection pipe 14, a pipe attachment nut 15, and a pinching attachment metal fitting 16.

As shown in Fig. 1, the casing 11 includes a casing main body 111 to which a substantially rectangular parallelepiped portion is connected in a longitudinal direction below a substantially elongated cigar-shaped portion extending from the base end side R to the tip side F, a grip portion 112 formed integrally with the base end side R of a substantially rectangular parallelepiped portion of the casing main body 111, and an extension portion 113 disposed on the base end side R of the casing main body 111 and the grip portion 112 and extending toward the base end side R, and is formed in substantially a pistol shape.

In addition, for example, the casing 11 is configured to be split into left and right sides in a cross section shown in Fig. 1, formed of a plastic resin having a substantially fixed thickness, and having a boss, an attachment hole, and the like, appropriately disposed thereon.

In addition, the casing 11 has an internal space in which the casing main body 111 and the grip portion 112 are connected integrally, and the base plate 12, the pipe attachment member 13, the pinching arm opening/closing drive portion 30, the pinching arm pivotal drive portion 40, and the like, are disposed in the internal space. In addition, the left and right casings 11 are assembled integrally on the attachment portions 11A, 111B and 11C by attachment bolts. In addition, the attachment hole 11H is formed in a tip portion of the casing 11.

As shown in Fig. 1, the grip portion 112 is formed in a substantially rectangular parallelepiped shape, and formed at an intersection angle θ = about 75° with respect to a longitudinal direction of the casing main body 111 (a direction along an axis line of the protection pipe 14).

Thus, an operator grasps the grip portion 112, and the pinching arm opening/closing drive portion 30 and the pinching arm pivotal drive portion 40 are operated.

For example, it is preferable that the intersection angle θ of the grip portion 112 with respect to the longitudinal direction of the casing main body 111 be formed at 45° to 90° for a stable grasping operation and pivotal operation, and it is more preferable that the intersection angle θ = 60° to 85°.

Further, the intersection angle θ may be arbitrarily set, and the intersection angle may be set to less than 45°.

As shown in Fig. 1, for example, the base plate 12 has a rectangular portion formed in a substantially rectangular shape, and a guide groove 12G is formed from the base end side R toward the base end side F of the rectangular portion.

In addition, for example, the base plate 12 is constituted by a pair of left and right base plates, an interval adjustment member such as a sleeve or the like is sandwiched between the left and right base plates 12, and thus, the pinching arm opening/closing drive portion 30 and the pinching arm pivotal drive portion 40 are disposed at a fixed interval and sandwiched from left and right sides.

In addition, a bracket extending toward the base end side R is connected to the base end side R of the substantially rectangular portion of the base plate 12 on the right side.

Then, for example, the base plate 12 is attached to the attachment portion 11A formed on the bracket and the casing 11 in an attachment portion 12A and an attachment portion 12B disposed on the rectangular portion.

As shown in Fig. 1, for example, the pipe attachment member 13 includes a skirt portion formed in a substantially truncated conical shape, and a multi-stage cylindrical portion extending from an apex portion of the skirt portion to the tip side F and having a pipe attachment hole formed therein to pass therethrough from the apex portion of the skirt portion in the longitudinal direction, and a male screw is formed on the tip side F of the multi-stage cylindrical portion.

Then, the pipe attachment member 13 can be attached to the tip side F of the casing 11 by correspondingly mounting the groove portion formed in the outer circumference of the base end side R of the multi-stage cylindrical portion on the edge portion of the attachment hole 11H of the casing 11.

As shown in Fig. 1, for example, the protection pipe 14 is constituted by a cylindrical stainless steel pipe, and configured to accommodate a part of the pinching arm opening/closing drive portion (the first drive part) 30 and some of the components of the pinching arm pivotal drive portion (the second drive part) 40 therein.

In addition, for example, it is appropriate that a guide member 14G such as a rod holder or the like be disposed in the protection pipe 14, and some of the components of the pinching arm opening/closing drive portion (the first drive part) 30 and the pinching arm pivotal drive portion (the second drive part) 40 be guided to the tip side F of the needle carrier body 10 from the casing 11.

As a result, the protection pipe 14 is configured to prevent the pinching arm opening/closing drive portion 30 and the pinching arm pivotal drive portion 40 from coming in contact with the biological tissue and minimize occurrence of operation errors or damage to them due to an external force.

Further, a type and a position of the guide member 14G may be arbitrarily set, and for example, may be more appropriately disposed on both of the tip side F and the base end side R of the protection pipe 14.

For example, the pipe attachment nut 15 has a multi-stage hole, and a female screw corresponding to the male screw of the pipe attachment member 13 is formed on an inner circumferential surface of the base end side R of the multi-stage hole. In addition, the tip side F of the multi-stage hole is formed to have an inner diameter corresponding to an outer diameter of the protection pipe 14.

Then, the pipe attachment member 13 can be held in the protection pipe 14 and the protection pipe 14 can be accurately and reliably attached to the casing 11 by inserting the protection pipe 14 into the pipe attachment hole of the pipe attachment member 13 and mounting the pipe attachment nut 15 on the pipe attachment member 13.

As shown in Figs. 2, 3A and 3B, for example, the pinching attachment metal fitting 16 includes a base plate wall portion 161 having a through-hole formed along the base end side R from the tip side F formed in a disk shape, a first support wall portion 162, a second support wall portion 163, and a cylindrical portion 164 connected to the base end side R of the base plate wall portion 161.

The first support wall portion 162 is formed from a left end of the base plate wall portion 161 to the tip side F in the longitudinal direction. In addition, a pivoting shaft receiving hole 16H corresponding to the second axis line O2 is formed in the first support wall portion 162.

The second support wall portion 163 is formed from a right end of the base plate wall portion 161 toward the tip side F in the longitudinal direction. In addition, the pivoting shaft receiving hole 16H corresponding to the second axis line O2 is formed in the second support wall portion 163.

In addition, the first support wall portion 162 and the second support wall portion 163 are formed to face each other, and a concave portion 16U is formed between the first support wall portion 162 and the second support wall portion 163.

Next, the pinching attachment (the grasping member) 20 according to the first embodiment will be described with reference to Figs. 2 to 7B.

As shown in Figs. 2, 3A and 3B, for example, the pinching attachment (the grasping member) 20 includes the pinching arm member 21, the pinching arm holding portion 24, a pinching operation receiving lever 25, a return spring (a pinching arm returning means) 26, and the support shaft 20J.

For example, the pinching arm member 21 includes the left pinching arm member 21L and the right pinching arm member 21R.

Then, the left pinching arm member 21L and the right pinching arm member 21R are relatively pivotable around the first axis line O1.

In addition, in the pinching arm member 21, the pinching portion 22 is formed on the tip side F in the longitudinal direction.

The pinching portion 22 includes a pinching protrusion (a pinching portion) 22L and a pinching protrusion (a pinching portion) 22R. In addition, the pinching protrusion 22L and the pinching protrusion 22R are configured such that the apex portion of the pinching protrusion 22L and the apex portion of the pinching protrusion 22R can face each other in the longitudinal direction (from the base end side R to the tip side F).

For example, a V-shaped pinching concave portion 23L (23) is formed between the pinching protrusion (the pinching portion) 22L and the pinching protrusion (the pinching portion) 22L, which are adjacent to each other, in a direction crossing (for example, perpendicular to) the longitudinal direction.

In addition, for example, a V-shaped pinching concave portion 23R (23) is formed between the pinching protrusion (the pinching portion) 22R and the pinching protrusion (the pinching portion) 22R, which are adjacent to each other, in a direction crossing (for example, perpendicular to) the longitudinal direction.

Then, the pinching protrusion 22L and the pinching protrusion 22R approach each other to pinch the object, and the pinching protrusion 22L and the pinching protrusion 22R are separated to release the object.

In the left pinching arm member 21L, the pinching operation receiving lever 25 is connected to the base end side R, and a pivoting shaft receiving hole (not shown) is formed between the left pinching arm member 21L and the pinching operation receiving lever 25 in the longitudinal direction.

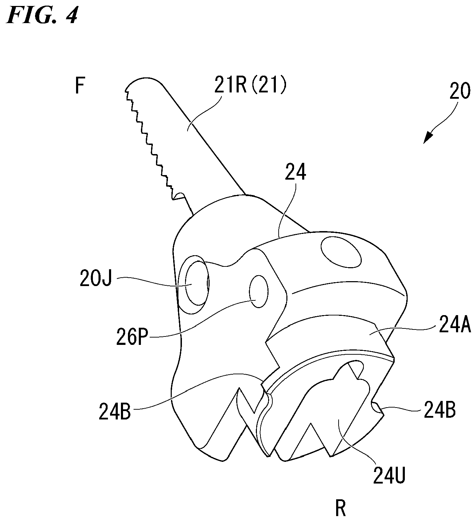

Next, a schematic configuration of the pinching arm holding portion 24 will be described with reference to Fig. 4.

Fig. 4 is a perspective view of a pinching attachment, which is a view explaining a schematic configuration of an attachment relationship between the pinching attachment and the grasping member pivoting portion according to the first embodiment.

As shown in Fig. 4, for example, the pinching arm holding portion 24 is disposed on the base end side R of the right pinching arm member 21R, and the right pinching arm member 21R and the pinching arm holding portion 24 are formed integrally with each other.

In addition, a through-hole 24U is formed in the pinching arm holding portion 24 from the base end side R toward the tip side F, a left side when seen from the base end side R is open and the upper sidewall portion and the lower sidewall portion face each other to form a concave portion on the tip side F of the through-hole 24U, and the left pinching arm member 21L is accommodated in the concave portion.

In addition, a through-hole into which the support shaft 20J corresponding to the first axis line O1 is fitted is formed in an upper sidewall portion and a lower sidewall of the pinching arm holding portion 24.

Then, the support shaft 20J is inserted into a pivoting shaft receiving hole (not shown) of the left pinching arm member 21L, and the left pinching arm member 21L is pivotably supported by the pinching arm holding portion 24 about the first axis line O1.

In the embodiment, the right pinching arm member 21R constitutes a fixed pinching arm member, and the left pinching arm member 21L constitutes a movable pinching arm member.

Then, since the left pinching arm member 21L is pivoted about the first axis line O1, the pinching protrusion 22L and the pinching protrusion 22R approach or are separated from each other to pinch the object.

In addition, the pinching arm holding portion 24 has an attachment boss 24A formed on the base end side R.

In addition, two flange portions (second engaging portions) 24B extending outward are formed in a part of the outer circumferential edge portion of the base end side R of the attachment boss 24A.

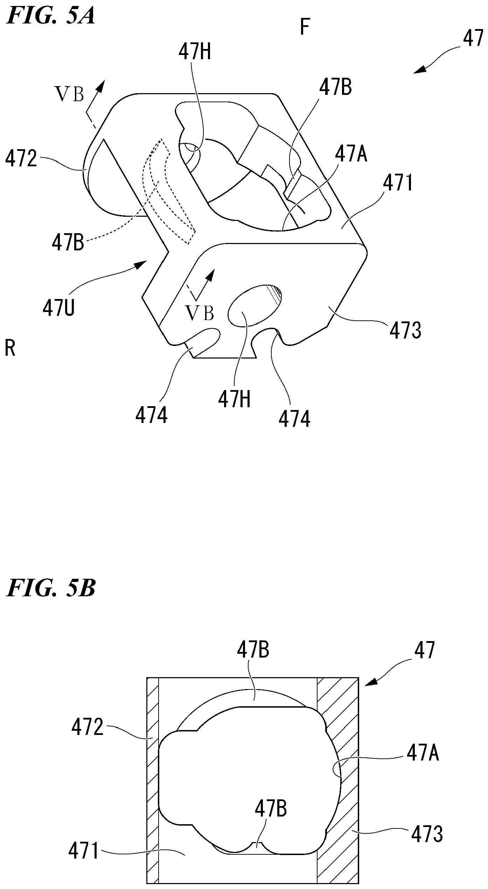

Here, a schematic configuration of a grasping member pivoting portion that constitutes a tip portion of the pinching arm pivotal drive portion (the second drive part) 40, which will be described below, will be described with reference to Figs. 5A and 5B.

Fig. 5A is a perspective view of a grasping member pivoting portion, which is a view explaining a schematic configuration of an attachment relationship between the pinching attachment and the grasping member pivoting portion. In addition, Fig. 5B is a cross-sectional view taken along arrow VB-VB in Fig. 5A when the grasping member pivoting portion is seen from the base end side in the attachment/detachment direction.

As shown in Figs. 5A and 5B, a grasping member pivoting portion 47 includes a base plate portion 471 formed in a substantially flat plate shape, a first support wall portion 472 extending from a left end of the base plate portion 471 to the base end side R, a second support wall portion 473 extending from a right end of the base plate portion 471 to the base end side R, and a pivoting control concave portion 474 formed in the second support wall portion 473, and a pivoting shaft receiving hole 47H is coaxially formed in the first support wall portion 472 and the second support wall portion 473.

A mounting hole 47A passing from the tip side F to the base end side R is formed in the base plate portion 471.

In addition, two flange portions (first engaging portions) 47B extending toward the inner circumferential side and corresponding to the two flange portions (the two second engaging portions) 24B are formed on the inner circumferential edge portion of the mounting hole 47A.

Then, the attachment boss 24A can be inserted into the mounting hole 47A of the grasping member pivoting portion 47 by being disposed such that the flange portion (the second engaging portion) 24B and the flange portion (the first engaging portion) 47B do not interfere with each other.

In addition, for example, the flange portion (the second engaging portion) 24B and the flange portion (the first engaging portion) 47B have screw-shaped tapered surfaces that are formed on surfaces thereof, which come in contact with each other.

Then, an axial force is generated in the attachment/detachment direction by tightening the flange portion (the second engaging portion) 24B and the flange portion (the first engaging portion) 47B to each other, and the flange portion (the second engaging portion) 24B and the flange portion (the first engaging portion) 47B are configured to be difficult to be removed by increasing a frictional force between the contact surfaces.

Further, whether such a screw-shaped tapered surface is formed on the flange portion 24B and the flange portion 47B can be arbitrarily set, and a stopper may have been accommodated therein already by a known means such as a ratchet or the like.

The return spring (the pinching arm returning means) 26 is constituted by, for example, a tensile spring (a coil spring).

In addition, as shown in Figs. 2, 3A and, 3B, a return spring 26 has a first end attached to a spring attachment pin 25P and a second end attached to a spring attachment pin 26P.

The spring attachment pin 26P is fixed to the pinching arm holding portion 24 through the through-hole 24U.

In addition, as shown in Figs. 3A and 3B, the first end of the return spring 26 is inserted into a spring insertion concave portion 25U formed in a side surface of the pinching operation receiving lever 25.

In addition, the spring attachment pin 25P is attached to the return spring 26 via a pin accommodating hole 25H formed from an upper surface of the pinching operation receiving lever 25 toward the spring insertion concave portion 25U.

Then, the pinching operation receiving lever 25 is pulled toward the spring attachment pin 26P such that the left pinching arm member 21L is biased to a side where the pinching protrusion 22L and the pinching protrusion 22R shown by arrow R12 are separated (opened).

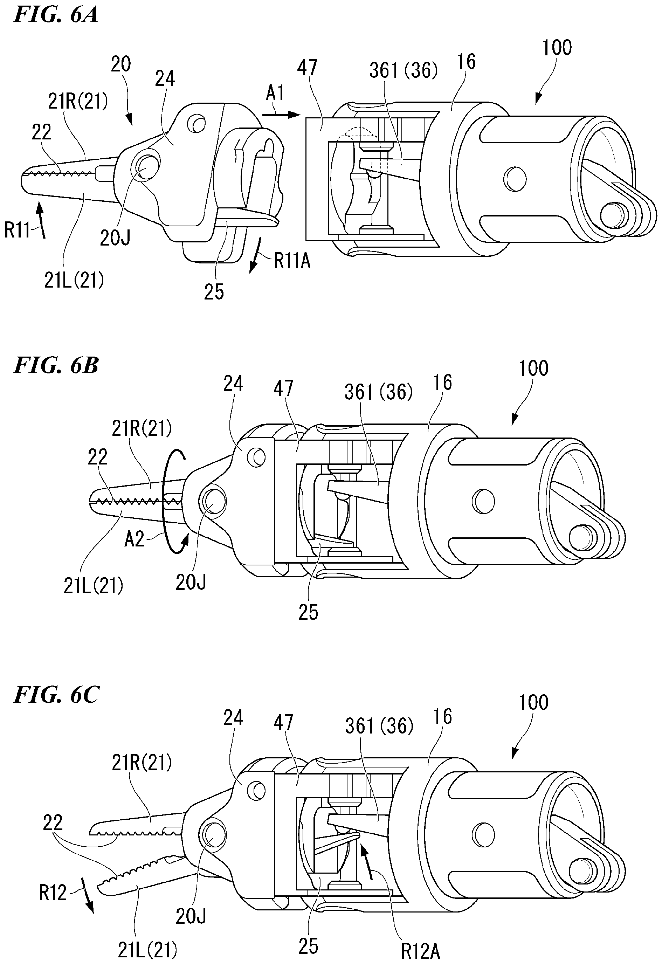

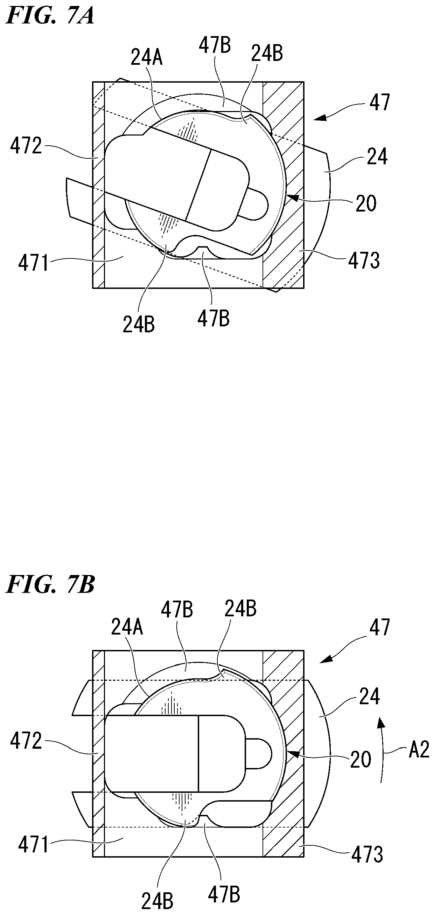

Next, a mounting procedure of the pinching attachment will be described with reference to Figs. 6A to 6C, 7A and 7B.

Figs. 6A to 6C are perspective views explaining a schematic configuration of the mounting procedure of the pinching attachment of the first embodiment, Fig. 6A is a view showing a state in which the pinching attachment is twisted to be inserted into the grasping member pivoting portion, Fig. 6B is a view showing a state in which the pinching attachment is mounted on the grasping member pivoting portion, and Fig. 6C is a view showing a state in which the pair of pinching arm members are opened to engage the pinching operation receiving lever with the pinching arm operating lever.

In addition, Figs. 7A and 7B are views explaining a schematic configuration of an attachment relationship between the pinching attachment and the grasping member pivoting portion, Fig. 7A is a view showing a state in which the pinching attachment is twisted with respect to the grasping member pivoting portion, and a state in which the pinching attachment is mounted on the grasping member pivoting portion from the base end side in the attachment/detachment direction, respectively. Here, the attachment/detachment direction indicates a direction shown by arrow A1 in Fig. 6A in which the pinching attachment 20 is inserted into the grasping member pivoting portion 47 and an opposite direction thereof, and the twisting direction indicates a direction shown by arrow A2 in Fig. 6B in which the inserted pinching attachment 20 is fastened to the grasping member pivoting portion 47 and an opposite direction thereof.
(1) First, as shown in Fig. 6A, the pinching attachment 20 is inserted into the grasping member pivoting portion 47 in the attachment/detachment direction shown by arrow A1.
   Here, as shown in Fig. 7A, the pinching attachment 20 is twisted by a predetermined angle around the axis line in the attachment/detachment direction, and the flange portion (the second engaging portion) 24B is disposed not to interfere with the flange portion (the first engaging portion) 47B.
   In addition, since the pinching arm member 21 is being pressed and closed by a finger or the like, the pinching operation receiving lever 25 is opened around the support shaft 20J in a direction shown by arrow R11A and disposed further outside than a pinching arm operating lever 36.
   Then, the attachment boss 24A is inserted into the mounting hole 47A of the grasping member pivoting portion 47, and the pinching attachment 20 is attached to the grasping member pivoting portion 47.
(2) Since the pinching attachment 20 is attached to the grasping member pivoting portion 47, as shown in Fig. 6B, the pinching attachment 20 is twisted in a direction shown by arrow A2.
   As a result, as shown in Fig. 7B, the flange portion (the second engaging portion) 24B of the pinching attachment 20 and the flange portion (the first engaging portion) 47B of the grasping member pivoting portion 47 overlap each other, and are not removed in the attachment/detachment direction.
   Then, since the pinching attachment 20 is further twisted in a direction as shown in arrow A2, the screw-shaped tapered surface of the flange portion (the second engaging portion) 24B and the screw-shaped tapered surface of the flange portion (the first engaging portion) 47B are more strongly screwed to increase an axial force.
   As a result, the pinching attachment 20 can be reliably mounted on the grasping member pivoting portion 47.
(3) Since the pinching attachment 20 is mounted on the grasping member pivoting portion 47, as shown in Fig. 6C, a finger or the like is released from the pinching arm member 21, and the left pinching arm member 21L is opened toward a side shown in arrow R21.

As a result, the pinching operation receiving lever 25 is pivoted around the support shaft 20J in a direction shown by arrow R12A to abut the pinching arm operating lever 36, and the pinching operation receiving lever 25 can be operated by the pinching arm operating lever 36.

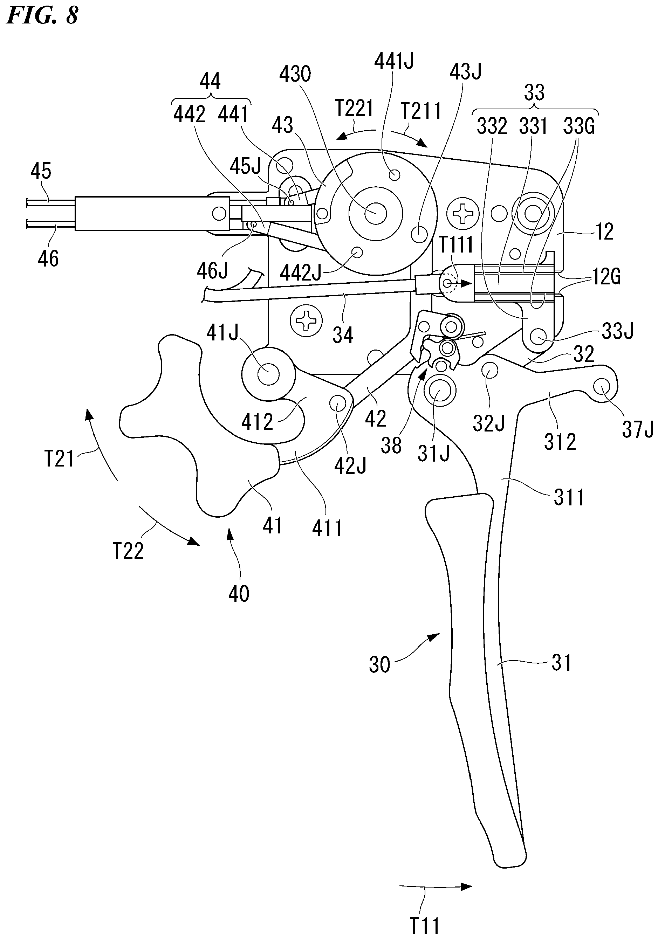

Next, the pinching arm opening/closing drive portion (the first drive part) 30 will be described with reference to Figs. 8 to 14B.

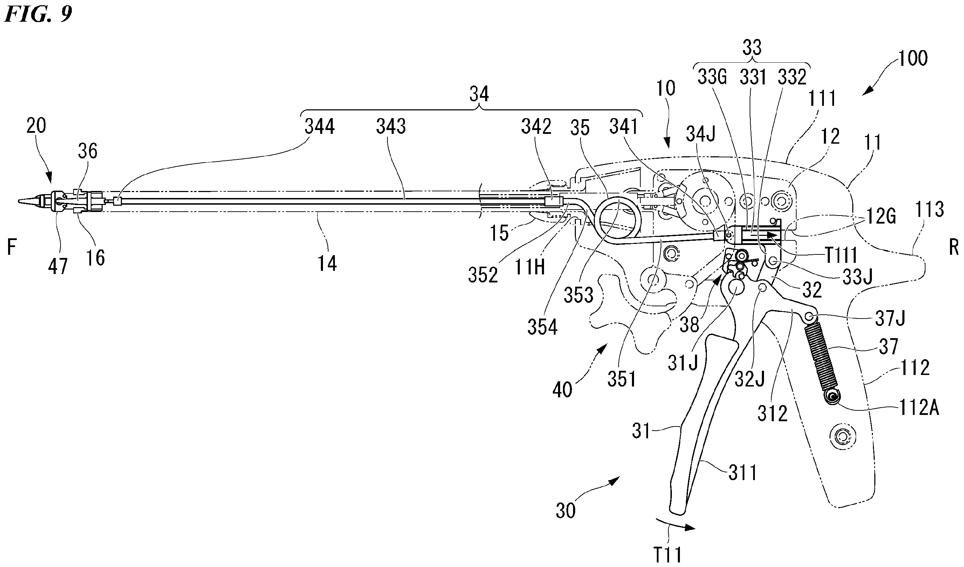

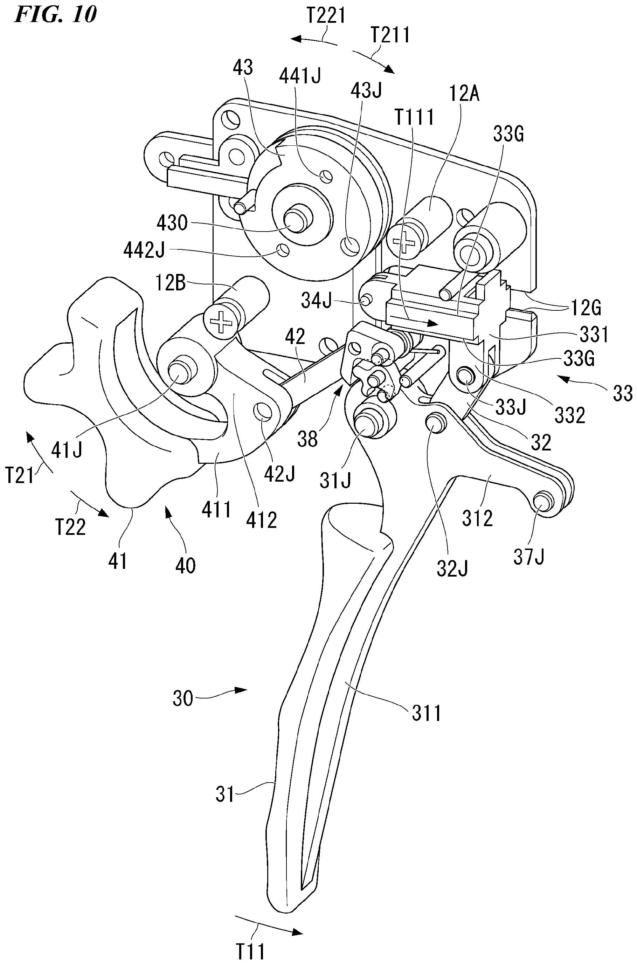

First, a schematic configuration of the pinching arm opening/closing drive portion 30 will be described with reference to Figs. 8 to 10. Fig. 8 is a longitudinal cross-sectional view explaining a schematic configuration of the pinching arm opening/closing drive portion 30 according to the first embodiment when seen in a side view, and Figs. 9 and 10 are a side view and a perspective view explaining major portions of the pinching arm opening/closing drive portion 30 and the pinching arm pivotal drive portion 40 in detail.

In Figs. 8 to 10, reference sign 31 designates a pinching operation lever (a pinching operation part), reference sign 32 designates a pinching operation transmission link, reference sign 33 designates a guide member, reference sign 34 designates a flexible transmission member, reference sign 35 designates an axial dimension expansion/contraction spring, reference sign 36 designates a pinching arm operating lever, reference sign 37 designates a pinching operation lever return spring, and reference sign 38 designates a pinching operation lock mechanism.

The pinching arm opening/closing drive portion (the first drive part) 30 has the pinching operation lever (the pinching operation part) 31 configured to cause the pair of pinching arm members 21 to perform a pinching operation, and a pinching operation transmission part configured to transmit the pinching operation from the pinching operation lever 31 to the pair of pinching arm members 21, and the pinching portion 22 is closed (pinched) by the pinching operation input to the pinching operation lever 31.

Specifically, as shown in Figs. 6 to 8, the pinching arm opening/closing drive portion (the first drive part) 30 includes the pinching operation lever (the pinching operation part) 31, the pinching operation transmission link 32, the guide member 33, the flexible transmission member 34, the pinching arm operating lever 36, the pinching operation return spring 37, and the pinching operation lock mechanism 38. In the embodiment, the pinching operation release mechanism is configured integrally with the pinching operation lock mechanism 38.

In addition, the pinching operation lever (the pinching operation part) 31, the pinching operation transmission link 32, the guide member 33, the flexible transmission member 34, and the pinching arm operating lever 36 are connected in this order.

In the embodiment, the pinching operation transmission part includes the pinching operation transmission link 32, the guide member 33, the flexible transmission member 34, and the pinching arm operating lever 36.

For example, the pinching operation lever (the pinching operation part) 31 includes a pinching lever main body 311 and a return spring lever 312, and is formed in a substantially L shape. In addition, the pinching operation lever 31 is supported on the base plate 12 by a support shaft 31J, and pivotable around the support shaft 31J.

In addition, when the pinching lever main body 311 is pivoted in an arrow T11 direction, the pinching operation can be input.

In addition, an arc-shaped circumferential surface is formed in the vicinity of the support shaft 31J of the pinching lever main body 311, and a pivoting gear portion (a gear portion) 383S constituted by steps that is rapidly displaced toward the inner circumferential side when pivoted in the arrow T11 direction is formed on the arc-shaped circumferential surface.

The pinching operation transmission link 32 is formed in, for example, an oval flat plate.

In addition, the pinching operation transmission link 32 has a first end pivotably connected to the pinching operation lever 31 by a support pin 32J and a second end pivotably connected to the guide member 33 by a support pin 33J.

Then, when the pinching operation is input from the pinching operation lever 31, the support pin 33J is moved toward the base end side R, and the pinching operation is transmitted to the guide member 33.

The guide member 33 includes a guide member main body 331 and a bracket 332.

A guide portion 33G is formed on the guide member main body 331 from the tip side F toward the base end side R, and the guide portion 33G is slidably disposed in the guide groove 12G of the base plate 12.

The bracket 332 is connected to the pinching operation transmission link 32 by the support pin 33J, and the pinching operation input from the pinching operation lever 31 is transmitted to the guide member 33.

As a result, the guide member main body 331 is slid along the guide groove 12G in an arrow Till direction by the pinching operation input from the pinching operation lever 31.

For example, the flexible transmission member 34 has a first end pivotably connected to a tip portion of the guide member 33 by a support pin 34J and a second end connected to the pinching arm operating lever 36. Then, the pinching operation input to the guide member 33 is transmitted to the pinching arm operating lever 36.

In addition, as shown in Fig. 9, the flexible transmission member 34 includes a first transmission part 341, a second transmission part 342, a third transmission part 343, a fourth transmission part 344, and the axial dimension expansion/contraction spring 35, and the axial dimension expansion/contraction spring 35 is disposed between the first transmission part 341 and the second transmission part 342.

In addition, in the embodiment, for example, the second transmission part 342 and the third transmission part 343 are disposed on the same line.

In addition, the flexible transmission member 34 is formed by a linear member such as a titanium alloy.

Further, the flexible transmission member 34 may be formed by a corrosion resistant metal having elasticity such as stainless steel (for example, SUS304/SUS304-CSP, SUS630, or the like) instead of the titanium alloy through rolling, heat treatment, or the like.

In addition, the flexible transmission member 34 may be formed of a plurality of different materials.

In the embodiment, the first transmission part 341, the second transmission part 342, the third transmission part 343 and the fourth transmission part 344 are hard members, and a longitudinal dimension of the flexible transmission member 34 (a widthwise dimension of the first transmission part 341 and the fourth transmission part 344) is expanded and contracted by the axial dimension expansion/contraction spring 35.

The first transmission part 341 has a first end connected to a tip portion of the guide member 33 by the support pin 34J and a second end joined to the base end side R of the axial dimension expansion/contraction spring 35.

Further, when the first transmission part 341, the axial dimension expansion/contraction spring 35, the second transmission part 342, the third transmission part 343 and the fourth transmission part 344 are joined to configure the flexible transmission member 34, for example, laser welding is appropriately used.

The second transmission part 342 has a first end joined to the tip side F of the axial dimension expansion/contraction spring 35 and a second end joined to the third transmission part 343, and the pinching operation is transmitted from the axial dimension expansion/contraction spring 35 to the third transmission part 343.

The third transmission part 343 is an elongated rod member, the first end is joined to the second transmission part 342, and the second end is joined to the fourth transmission part 344. Then, displacement of the second transmission part 342 is transmitted to the third transmission part 343.

The fourth transmission part 344 has a first end side joined to the third transmission part 343, and a chevron clevis is formed on the first end side. Then, the chevron clevis is inserted into the pinching arm operating lever 36 and pivotably connected thereto by a support shaft 36J.

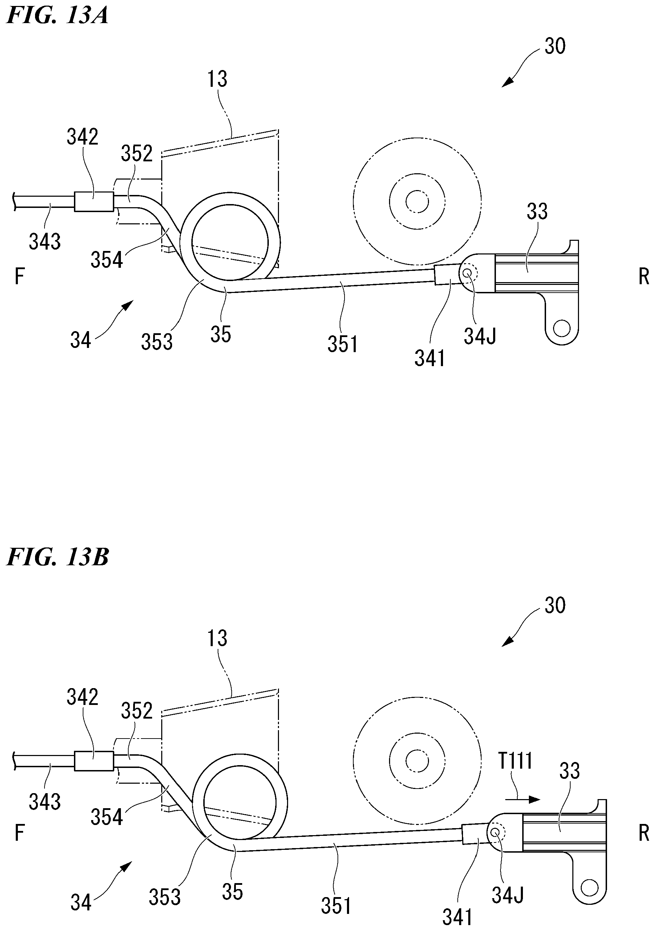

In addition, as shown in Figs. 9, 13A and 13B, for example, the axial dimension expansion/contraction spring 35 includes a first end portion 351 disposed on the first end side, a second end portion 352 disposed on the second end side, a winding portion (a circumferential portion) 353 disposed between the first end portion 351 and the second end portion 352 and on which a linear member is wound (for example, one turn or more) to form a substantially circular shape when seen in a side view, and a direction change portion 354 configured to change a direction in which the second end portion 352 extends between the winding portion 353 and the second end portion 352.

In addition, in the embodiment, the axial dimension expansion/contraction spring 35 is formed by, for example, a titanium alloy linear member (for example, about ϕ1.5 to 2 mm).

In the first end portion 351, for example, the first end side extends toward the base end side R and is connected to the first transmission part 341. In addition, the second end side is connected to the first end of the winding portion (the circumferential portion) 353.

The second end portion 352 is disposed at a position offset from the first end portion 351 when seen along, for example, an axis line, and the first end side is connected to the winding portion (the circumferential portion) 353 via the direction change portion 354.

In addition, the second end portion 352 extends toward a side opposite to the first end portion 351, and the second end side is connected to the second transmission part 342.

For example, the winding portion (the circumferential portion) 353 is formed by winding the titanium alloy linear member (for example, one turn or more).

The direction change portion 354 is configured in, for example, a curved shape (or a turn shape), the second end portion 352 is offset from the first end portion 351 extending from the winding portion (the circumferential portion) 353 in a winding direction, and the first end portion 351 and the second end portion 352 extend in opposite directions.

In the axial dimension expansion/contraction spring 35, according to the above-mentioned configuration, when a load in an axial direction (a tensile direction, a compression direction) is added between the first end portion 351 and the second end portion 352, a diameter of a circular shape of the winding portion 353 is contracted or expanded.

As a result, even when an excessive load is added between the first end portion 351 and the second end portion 352, displacement of the axial dimension expansion/contraction spring 35 in the axial direction can be absorbed.

Further, the number of turns of the winding portion 353 may be a plurality of times.

In addition, the circular shape that forms the winding portion 353 is not limited to when seen from the side view, and may be disposed to circumferentially surround the winding portion when seen in a plan view.

In addition, the winding portion 353 is not limited to a true circular shape and may be an oval shape, or the like.

For example, the pinching arm operating lever 36 includes a pinching operation output lever 361 and an input lever 362, and a bearing hole (not shown) is formed between the pinching operation output lever 361 and the input lever 362.

In addition, a U-shaped clevis is formed on the input lever 362, and a chevron clevis of the fourth transmission part 344 of the flexible transmission member 34 is mounted on the U-shaped clevis and pivotably connected thereto by the support shaft 36J.

In addition, a bearing hole (not shown) formed in the pinching arm operating lever 36, a through-hole 164H formed in the cylindrical portion 164 of the pinching attachment metal fitting 16 and a hole (not shown) formed in the protection pipe 14 overlap each other and a support shaft 37 is fitted into these holes, and thus, the pinching arm operating lever 36 is configured to be pivotable around an axis line O3 parallel to the first axis line O1.

Then, since an arm operating lever 36 is pivoted around the axis line O3 by the pinching operation, the arm operating lever causes the left pinching arm member 21L to perform the pinching operation via the pinching operation output lever 361.

The pinching operation return spring 37 is constituted by, for example, a coil spring, the first end is connected to the return spring lever 312 of the pinching operation lever 31 by a support pin 37J, and the second end is connected to an attachment boss 112A in the grip portion 112.

Then, the pinching operation lever 31 is configured to be biased in an orientation opposite to the arrow T11 direction.

Further, instead of the pinching operation return spring 37, for example, a spiral spring, a torsion spring, or the like, may be used.

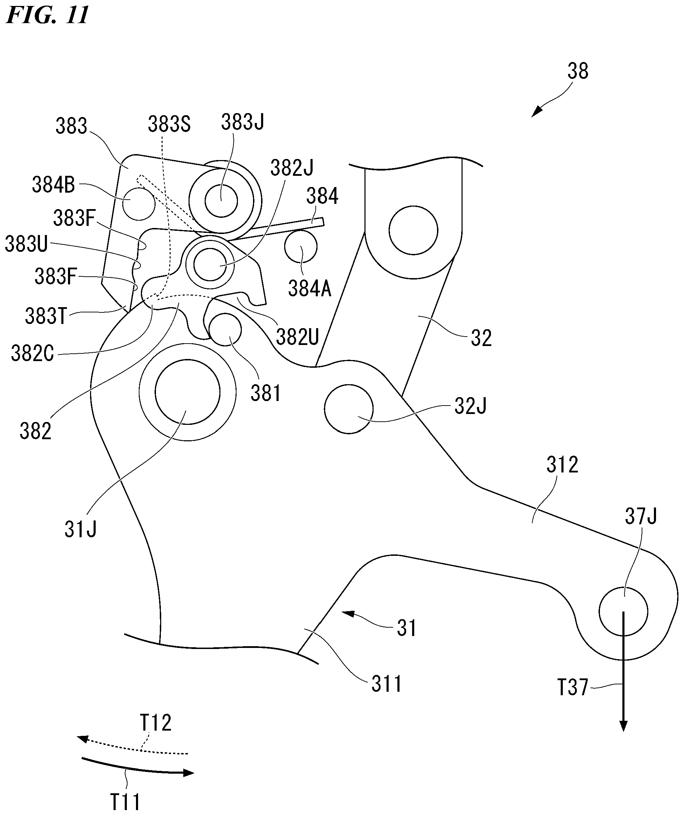

Next, a pinching operation lock mechanism will be described with reference to Fig. 11.

Fig. 11 is a schematic configuration view showing a schematic configuration of the pinching operation lock mechanism of the pinching arm opening/closing drive portion according to the first embodiment when seen in a side view. In Fig. 11, reference sign 381 designates a lock mechanism drive pin, reference sign 382 designates a ratchet drive cam, reference sign 383 designates a ratchet lever, and reference sign 384 designates a ratchet lever biasing spring.

As shown in Fig. 11, for example, the pinching operation lock mechanism 38 includes the lock mechanism drive pin 381, the ratchet drive cam 382, the ratchet lever 383, and the ratchet lever biasing spring 384.

Then, an operation (displacement) due to the pinching operation input from the pinching operation lever (the pinching operation part) 31 is locked at a predetermined position, and a pinching state of an object by the pinching portion 22 is held.

For example, the lock mechanism drive pin 381 is constituted by a pin standing on the pinching operation lever 31, and turns around the support shaft 31J together with pivotal movement of the pinching operation lever 31 by the pinching operation.

As shown in Fig. 11, for example, the ratchet drive cam 382 is formed in a flat plate shape having a substantially pentagonal appearance, and includes a support shaft 382J standing upward from the vicinity of one vertical angle, a drive pin receiving concave portion 382U, and a ratchet locking protrusion 382C.

Then, the ratchet drive cam can pivot (swing) around the support shaft 382J.

As shown in Fig. 11, the drive pin receiving concave portion 382U is disposed at an opposite side of the support shaft 382J, constituted by a concave portion formed to have a width having a larger dimension than that of a diameter of the lock mechanism drive pin 381, and has a play with respect to the lock mechanism drive pin 381.

As a result, in the ratchet drive cam 382, the ratchet drive cam 382 is pivotable (swingable) around the support shaft 382J except when pressed against the lock mechanism drive pin 381.

The ratchet locking protrusion 382C is disposed on the ratchet drive cam 382 on the side of the ratchet lever 383, and an apex portion is formed to protrude in a substantially semi-circular shape.

For example, the ratchet lever 383 is constituted by a substantially L-shaped flat plate, and includes a support shaft 383J standing from the vicinity of an end portion of one side of the L shape, a flat portion 383F formed on an edge portion of the ratchet drive cam 382 of the other side of the L shape, an arc-shaped ratchet locking concave portion 383U formed in the middle of the flat portion 383F, and a ratchet claw portion (a locking claw portion) 383T formed at a tip side of the other side of the L shape.

Then, the ratchet lever 383 is configured to pivot (swing) around the support shaft 383J.

In addition, the ratchet claw portion (the locking claw portion) 383T is configured to correspond to the pivoting gear portion (the gear portion) 383S of the pinching operation lever 31.

Then, since the ratchet claw portion (the locking claw portion) 383T is hooked to and engaged with the pivoting gear portion (the gear portion) 383S, the pinching operation lever 31 is not pivoted in a direction corresponding to a tensile direction T37 of the pinching operation return spring 37 (in Fig. 11, a direction shown by arrow T12). As a result, the pinching state (a closed state) of the pinching portion 22 is held.

Meanwhile, when engagement between the ratchet claw portion (the locking claw portion) 383T and the pivoting gear portion (the gear portion) 383S is removed, the pinching operation lever 31 is recovered in an arrow T12 direction while the pinching state (the closed state) of the pinching portion 22 is released.

The ratchet lever biasing spring 384 is constituted by, for example, a torsion spring.

Then, the ratchet lever biasing spring 384 is configured to be supported by a support pin 384A axially supported by the support shaft 383J and standing from the base plate and a support pin 384B standing from the ratchet lever 383, and to bias the ratchet lever 383 such that the ratchet claw portion (the locking claw portion) 383T is disposed on the side of the pivoting gear portion (the gear portion) 383S.

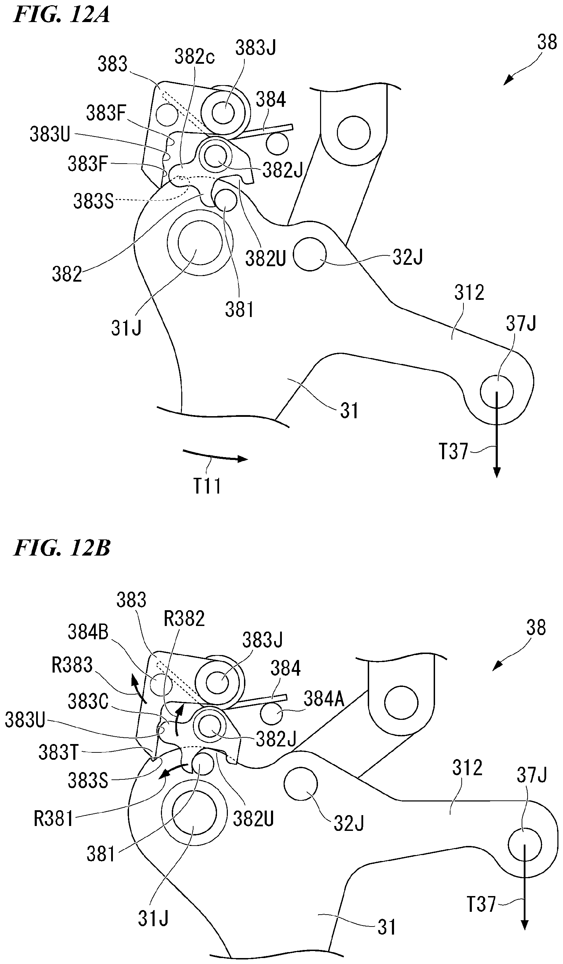

Next, actions of the pinching arm opening/closing drive portion 30 and the pinching operation lock mechanism 38 will be described with reference to Figs. 12A to 13B.

Figs. 12A and 12B are views explaining actions of the pinching arm opening/closing drive portion and the pinching operation lock mechanism according to the first embodiment, Fig. 12A shows a state before the pinching operation is started, Fig. 12B shows a state when the pinching operation is terminated. In addition, Fig. 13A is a schematic configuration view showing the axial dimension expansion/contraction spring in a state in which the pinching arm member is open, and Fig. 13B is a schematic configuration view showing the axial dimension expansion/contraction spring in a state in which the pinching arm member is closed.

### <Pinching operation>

(1) First, in a state in which the pinching operation is not performed, the pinching operation lever 31 is disposed at a position in the circumferential direction about the support shaft 31J shown in Fig. 12A.
   Here, the lock mechanism drive pin 381 is in a free state in the drive pin receiving concave portion 382U of the ratchet drive cam 382, and the ratchet locking protrusion 382C is not engaged with the ratchet locking concave portion 383U. In addition, the ratchet claw portion (the locking claw portion) 383T is not engaged with the pivoting gear portion (the gear portion) 383S and disposed on a circumferential surface of the pinching operation lever 31.
   Here, as shown in Fig. 13A, the axial dimension expansion/contraction spring 35 is in a state in which the pinching operation is not input, and a load (a tensile force) is not added between the first end portion 351 and first end portion352.
   In this case, the winding portion (the circumferential portion) 353 is in a free state, and a circular shape that forms the winding portion 353 has a large diameter. As a result, the first transmission part 341 and the second transmission part 342 are in close proximity, and the pinching arm member 21 is in a state in which the pinching portion 22 is open.
(2) Next, as shown in Fig. 12A, the pinching operation lever 31 for a pinching operation is pivoted in the arrow T11 direction.
   When the pinching operation lever 31 is pivoted in the arrow T11 direction, as shown in Fig. 12B, the lock mechanism drive pin 381 abuts the drive pin receiving concave portion 382U and the ratchet drive cam 382 is pivoted in an arrow R382 direction.
   When the ratchet drive cam 382 is pivoted in the arrow R382 direction, the ratchet locking protrusion 382C pivots the ratchet lever 383 in an arrow R383 direction, and a ratchet locking protrusion 38C is engaged with the ratchet locking concave portion 383U and the ratchet lever 383 is stopped at a predetermined position in the circumferential direction.
   In addition, when the ratchet locking protrusion 38C is engaged with the ratchet locking concave portion 383U, the ratchet lever 383 is slightly pivoted in a direction opposite to the arrow R383 direction by the ratchet lever biasing spring 384, the ratchet claw portion (the locking claw portion) 383T is engaged with the pivoting gear portion (the gear portion) 383S, and the pinching operation lever 31 is locked.

When the guide member 33 is moved in an arrow T111 direction and the pinching operation is input via the first transmission part 341, as shown in Fig. 13B, the circular shape that forms the winding portion 353 of the axial dimension expansion/contraction spring 35 is reduced in diameter, and the second transmission part 342 is biased to the base end side R. Then, the biasing force is transmitted to the pinching arm operating lever 36 via the third transmission part 343 and the fourth transmission part 344, and the left pinching arm member 21L is closed.

Here, in the axial dimension expansion/contraction spring 3, since the circular shape that forms the winding portion 353 is reduced in diameter, application of an excessive force from the pinching operation lever 31 to the pinching arm member 21 due to the pinching operation is minimized.

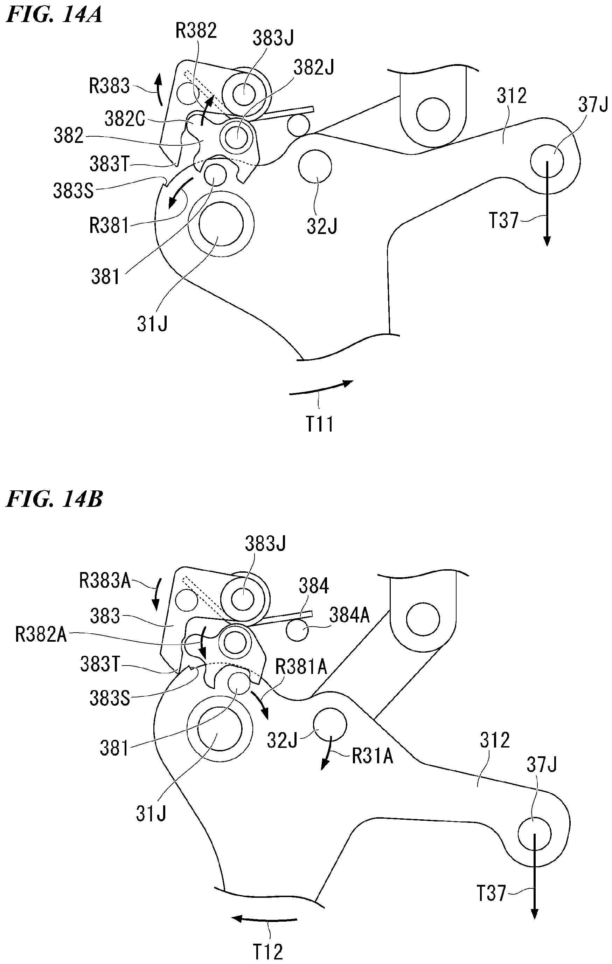

Next, release of the pinching operation will be described with reference to Figs. 14A and 14B.

Figs. 14A and 14B are views explaining release of the pinching operation lock mechanism according to the first embodiment, Fig. 14A is a schematic configuration view showing a state in which release of the pinching operation lock mechanism is started, and Fig. 14B is a schematic configuration view showing a state in which the pinching operation lock mechanism is released.

### <Pinching release operation>

(1) First, as shown in Fig. 14A, the pinching operation lever 31 is slightly pivoted in the arrow T11 direction.
   When the pinching operation lever 31 is slightly pivoted in the arrow T11 direction, the lock mechanism drive pin 381 is pivoted in a direction shown by arrow R381, and the ratchet drive cam 382 is pivoted in the arrow R382 direction. As a result, the ratchet locking protrusion 38C escapes from the drive pin receiving concave portion 382U in the arrow R382 direction, and the ratchet lever 383 is pivoted in the arrow R383 direction.
   As a result, engagement between the ratchet claw portion (the locking claw portion) 383T and the pivoting gear portion (the gear portion) 383S is released.
(2) When engagement between the ratchet claw portion (the locking claw portion) 383T and the pivoting gear portion (the gear portion) 383S is released, as shown in Fig. 14B, the lock mechanism drive pin 381 abuts the drive pin receiving concave portion 382U to be pivoted in an arrow R381A direction.

In addition, the pinching operation lever 31 is pivoted in the arrow T12 direction by the tensile direction T37 of the pinching operation return spring 37 to return to an original position.

Next, the pinching arm pivotal drive portion will be described with reference to Figs. 7, 8, 15 and 16.

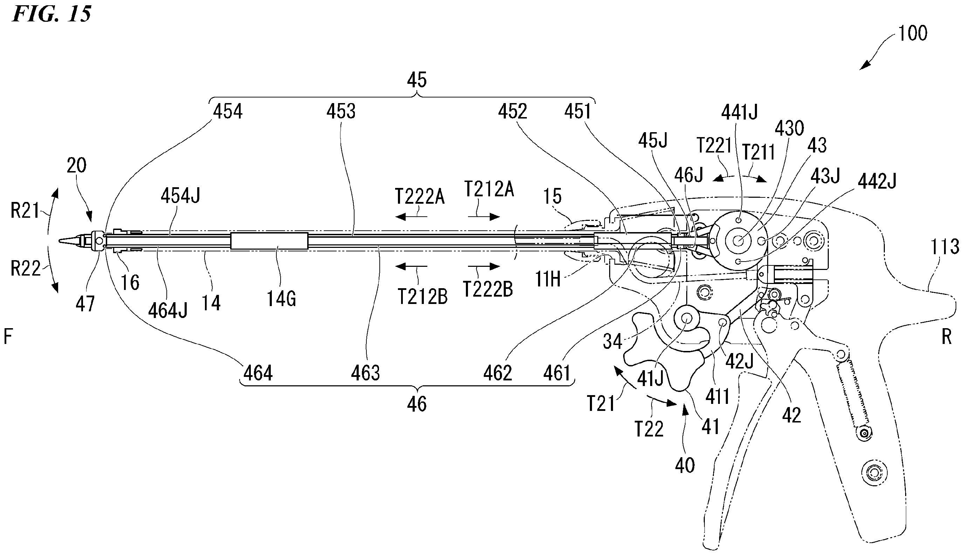

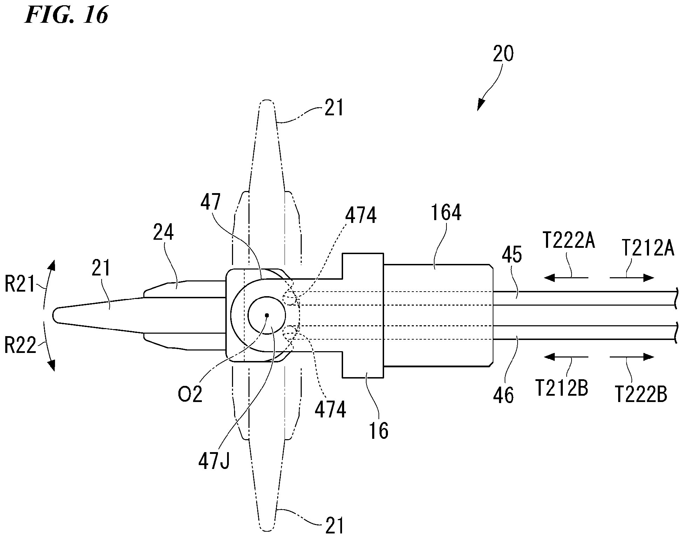

Fig. 15 is a longitudinal cross-sectional view explaining a schematic configuration of the pinching arm pivotal drive portion according to the first embodiment when seen in a side view, and Fig. 16 is a schematic configuration view explaining an action of the pinching arm pivotal drive portion when seen in a side view. In Figs. 15 and 16, reference sign 40 designates a pinching arm pivotal drive portion (a second drive part), reference sign 41 designates a pivotal operation wheel (a pivotal operation part), reference sign 42 designates a pivotal operation transmission link, reference sign 43 designates a pivoting control cam, reference sign 44 designates a transmission link, reference sign 45 designates a first transmission rod, reference sign 46 designates a second transmission rod, and reference sign 47 designates a grasping member pivoting portion.

The pinching arm pivotal drive portion (the second drive part) 40 includes, as shown in Figs. 15, 7 and 8, for example, the pivotal operation wheel (the pivotal operation part) 41, the pivotal operation transmission link 42, the pivoting control cam 43, the transmission link 44, the first transmission member 45, the second transmission member 46, and the grasping member pivoting portion 47 configured to pivot the pinching arm member 21 around the second axis line O2 using a pivotal operation.

The pivotal operation wheel (the pivotal operation part) 41 includes a wheel main body portion 411 formed in an arc shape, and a lever portion 412 formed on a first end of the wheel main body portion 411.

The pivotal operation wheel 41 is supported by a support shaft 41J and pivotable around the support shaft 41J. In addition, a finger receiving member is mounted on an outer circumference of the wheel main body portion 411 such that a finger is easily caught.

Then, the wheel main body portion 411 is configured to be pivoted around the support shaft 41J in directions of arrows T21 and T22 by the pivotal operation.

The pivotal operation transmission link 42 is constituted by an elongated flat plate curved at a central portion having substantially a "<" shape.

In addition, the pivotal operation transmission link 42 is pivotably connected to an outer circumferential portion of the lever portion 412 of the pivotal operation wheel 41 around the support pin 42J by a support pin 42J.

In addition, the pivotal operation transmission link 42 is pivotably connected to an outer circumferential portion of the pivoting control cam 43 around the support shaft 43J by a support shaft 43J.

Then, the pivotal operation input from the pivotal operation wheel 41 is transmitted to the pivoting control cam 43.

The pivoting control cam 43 is formed in a substantially circular plate shape, and pivotably supported on the base plate 12 by a support shaft 430.

In addition, in the pivoting control cam 43, the support shaft 43J, a support shaft 441J and a support shaft 442J are disposed on the outer circumferential portion.

In addition, the support shaft 441J and the support shaft 442J are disposed at an interval of about 90° with the support shaft 43J sandwiched therebetween in the circumferential direction.

Then, the pivoting control cam 43 is pivoted around the support shaft 430J by the pivotal operation input from the pivotal operation wheel 41 via the pivotal operation transmission link 42, and the pivotal operation is transmitted to the transmission link 44. Specifically, when the pivotal operation wheel 41 is pivoted in the arrow T21 direction, the pivoting control cam 43 is pivoted in an arrow T211 direction, and when the pivotal operation wheel 41 is pivoted in the arrow T22 direction, the pivoting control cam 43 is pivoted in an arrow T221 direction.

The transmission link 44 includes a first transmission link 441 and a second transmission link 442.

When the first transmission link 441 and the second transmission link 442 are seen from a side view, the first transmission link 441 is disposed above the second transmission link 442.

The first transmission link 441 is constituted by an oval flat plate. In addition, the first transmission link 441 has a first end pivotably connected to the pivoting control cam 43 by the support shaft 441J and a second end pivotably connected to the first transmission member 45 by a support shaft 45J.

Then, the first transmission link 441 transmits a pivotal operation from the pivoting control cam 43 to the first transmission member 45.

The second transmission link 442 is constituted by an oval flat plate. In addition, the second transmission link 442 has a first end pivotably connected to the pivoting control cam 43 by the support shaft 442J and a second end pivotably connected to the second transmission member 46 by a support shaft 46J.

Then, the second transmission link 442 transmits a pivotal operation from the pivoting control cam 43 to the second transmission member 46.

The first transmission member 45 includes a first member 451, a second member 452, a third member 453 and a fourth member 454, and the first member 451, the second member 452, the third member 453 and the fourth member 454 are disposed from the base end side R toward the tip side F in this order.

The first member 451 has a first end pivotably connected to the first transmission link 441 by the support shaft 45J and a male screw is formed on an outer circumference of a second end (the tip side F).

The third member 453 has a first end (the base end side R) having a male screw formed on an outer circumference thereof and a second end connected to the fourth member 454. Further, the male screw of the third member 453 is formed in an opposite direction (a reverse screw) of the male screw of the first member 451.

The second member 452 is formed in, for example, a cylindrical shape, and constituted by a male screw of the first member 451 on an inner side, and a turnbuckle having a female screw corresponding to the male screw of the third member 453.

Then, since the second member 452 is pivoted around the axis line, the first member 451 and the third member 453 can approach or be separated from each other to adjust a length of the first transmission member 45.

Further, whether the second member 45 is the length adjustment member such as a turnbuckle or the like may be arbitrarily set, and may have a configuration in which a length adjustment function is not provided.

The fourth member 454 includes, for example, a transmission pin portion 454J pivotably connected to the grasping member pivoting portion 47, and a pivotal operation transmitted from the third member 453 is transmitted to the grasping member pivoting portion 47.

In the embodiment, the transmission pin portion 454J is constituted by a needle bearing or the like elongated along the second axis line O2, and offset from a laterally central portion of the protection pipe 14 to be connected to the grasping member pivoting portion 47.

As a result, influence due to a decrease in dimension of the protection pipe 14 in a height direction at a peripheral portion in a widthwise direction is minimized.

The second transmission member 46 includes a first member 461, a second member 462, a third member 463 and a fourth member 464, and the first member 461, the second member 462, the third member 463 and the fourth member 464 are disposed from the base end side R toward the tip side F in this order.

The first member 461 has a first end pivotably connected to the second transmission link 442 by the support shaft 46J and a second end (the tip side F) having a male screw formed on an outer circumference thereof.

The third member 463 has a first end (the base end side R) having a male screw formed on an outer circumference thereof and a second end connected to the fourth member 464. Further, the male screw of the third member 463 is formed in an opposite direction (a reverse screw) of the male screw of the first member 461.

The second member 462 is formed in, for example, a cylindrical shape, and constituted by the male screw of the first member 461 on an inner side and a turnbuckle having a female screw corresponding to the male screw of the third member 463.

Then, the first member 461 and the third member 463 can approach or be separated from each other by pivoting the second member 462 to adjust a length of the second transmission member 46.

Further, whether the second member 46 is the length adjustment member such as a turnbuckle or the like can be arbitrarily set, and may have a configuration in which the length adjustment function is not provided.

The fourth member 464 includes, for example, a transmission pin portion 454J pivotably connected to the grasping member pivoting portion 47, and a pivotal operation transmitted from the third member 463 is transmitted to the grasping member pivoting portion 47.

In the embodiment, the transmission pin portion 464J is constituted by a needle bearing or the like elongated along the second axis line O2, and offset from a laterally central portion of the protection pipe 14 to be connected to the grasping member pivoting portion 47.

As described above, the grasping member pivoting portion 47 includes the base plate portion 471, the first support wall portion 472 extending from a left end of the base plate portion 471, the second support wall portion 473 extending from a right end of the base plate portion 471, and the pivoting control concave portion 474 formed in the second support wall portion 473, and the pivoting shaft receiving hole 47H is formed coaxially with the first support wall portion 472 and the second support wall portion 473.

For example, two pivoting control concave portions 474 are formed in an outer circumference of the second support wall portion 473.

Then, the transmission pin portion 454J of the fourth member 454 and the transmission pin portion 464J of the fourth member 464 are pivotably engaged with the pivoting control concave portions 474, respectively.

Cylindrical pins are joined to end portions of the transmission pin portion 454J of the fourth member 454 and the transmission pin portion 464J of the fourth member 464 on the tip side F in a direction perpendicular to major axes of the transmission pin portion 454J and the transmission pin portion 464J, and the pins are configured to engage with the pivoting control concave portion 474.

In addition, a concave portion 47U in which the pinching arm operating lever 36 is disposed is formed between the first support wall portion 472 and the second support wall portion 473.

In addition, the grasping member pivoting portion 47 is disposed in the concave portion 16U of the pinching attachment metal fitting 16, the pivoting shaft receiving hole 16H formed in the first support wall portion 162 of the pinching attachment metal fitting 16 and the pivoting shaft receiving hole 47H formed in the first support wall portion 472 of the grasping member pivoting portion 47 overlap each other such that a pivoting support shaft 47J is inserted therethrough, and thus, the pivoting support shaft 47J is pivotably connected to the pinching attachment metal fitting 16 around the second axis line O2.

Similarly, the pivoting shaft receiving hole 16H formed in the second support wall portion 163 and the pivoting shaft receiving hole 47H formed in the second support wall portion 473 overlap each other such that the pivoting support shaft 47J is inserted therethrough, and thus, the pivoting support shaft 47J is pivotably connected to the pinching attachment metal fitting 16 around the second axis line O2.

As a result, as shown in Fig. 16, when the pivotal operation wheel 41 is pivoted in the arrow T21 direction, the grasping member pivoting portion 47 is pivoted such that the first transmission member 45 is moved in an arrow T212A direction, the second transmission member 46 is moved in an arrow T212B direction, and the pinching arm member 21 is pivoted in the arrow R21 direction.

In addition, when the pivotal operation wheel 41 is pivoted in an arrow T22 direction, the grasping member pivoting portion 47 is pivoted such that the first transmission member 45 is moved in an arrow T222A direction, the second transmission member 46 is moved in an arrow T222B direction, and the pinching arm member 21 is pivoted in an arrow R22 direction.

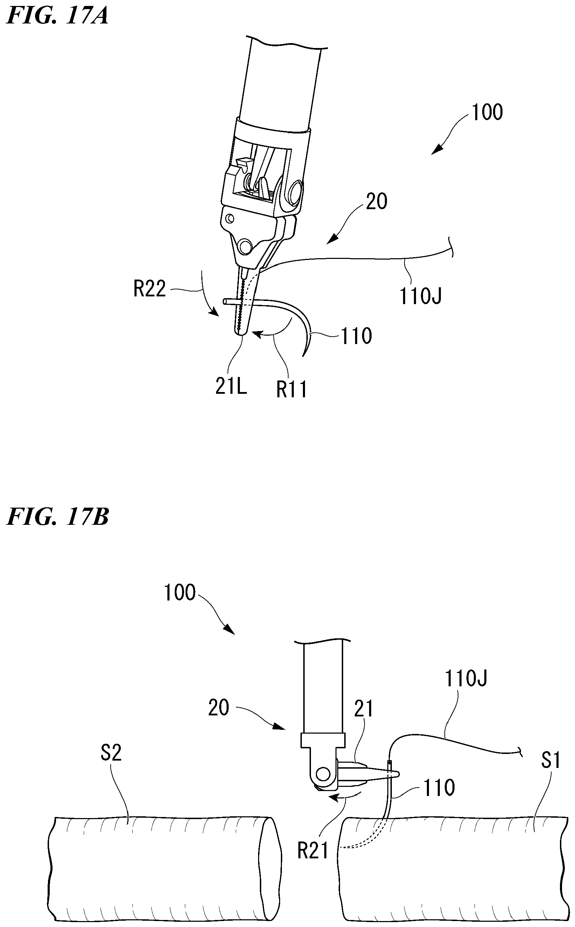

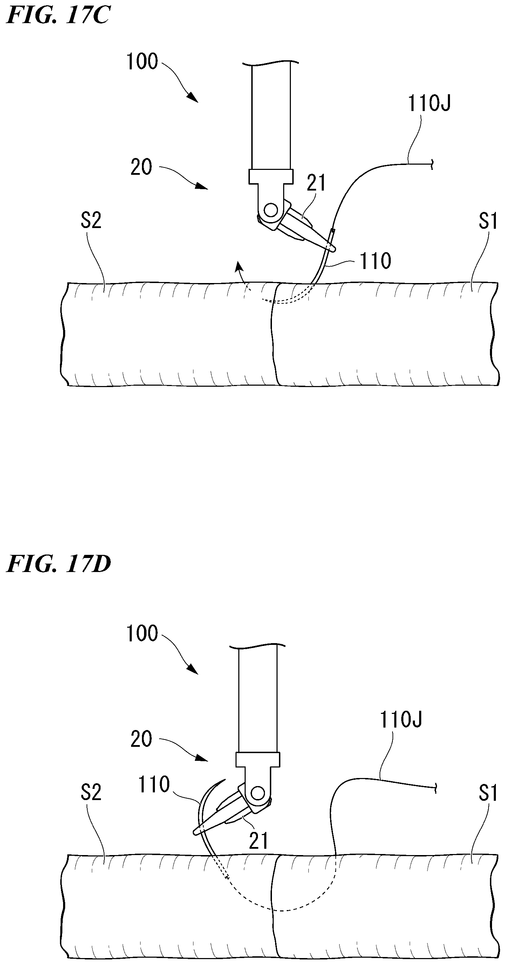

Next, handling of a suturing needle 110 by the needle carrier 100 will be described with reference to Figs. 17A to 17D. Figs. 17A to 17D are views explaining an example of an action of the needle carrier according to the first embodiment, Fig. 17A shows a state in which the suturing needle is pinched by the pinching arm member 21, Fig. 17B shows a state in which the suturing needle is pierced into a first biological tissue, Fig. 17C shows a state in which the first biological tissue and a second biological tissue are sutured, and Fig. 17D shows a state in which the suturing needle is changed after suturing. In the drawings, reference sign 110 designates a suturing needle, reference sign 110T designates a suturing thread, reference sign S1 designates a first biological tissue that is first pierced by the suturing needle 110, and reference sign S2 indicates a second biological tissue.

Further, the first biological tissue S1 and the second biological tissue S2 correspond to, for example, a blood vessel and the like.

(1) First, as shown in Fig. 17A, the left pinching arm member 21L is pivoted in the arrow R1 direction and the suturing needle 110 is pinched by the pinching arm member 21.
   Then, the pinching arm member 21 is pivoted in, for example, the arrow R22 direction.
(2) Next, as shown in Fig. 17B, end portions of the first biological tissue S1 and the second biological tissue S2 approach each other. Then, the pinching arm member 21 is moved such that the suturing needle 110 is disposed in the vicinity of the end portion of the first biological tissue S1.
   Then, the pinching arm member 21 is pivoted in the arrow R21 direction to handle the suturing needle 110, and the suturing needle 110 is pierced into the first biological tissue S1.
   A posture and a position of the needle carrier 100 with respect to the first biological tissue S1 are appropriately adjusted according to a situation of the handling of the needle.
(3) Next, as shown in Fig. 17C, the end portions of the first biological tissue S1 and the second biological tissue S2 are matched. Then, the suturing needle 110 passes through the first biological tissue S1, and the suturing needle 110 is handled from the first biological tissue S1 toward the second biological tissue S2.
(4) Next, as shown in Fig. 17D, since the suturing needle 110 passes through the first biological tissue S1 and the second biological tissue S2 and the tip portion of the suturing needle 110 protrudes from the second biological tissue S2, the pinching arm member 21 is opened once and the pinching arm member 21 is released from the suturing needle 110.

Then, the tip side of the suturing needle 110 is pinched again and the suturing needle 110 is pulled out of the second biological tissue S2, and the first biological tissue S1 and the second biological tissue S2 are connected by the suturing thread 110T.

Operations of the above-mentioned (1) to (4) are repeated, and the end portions of the first biological tissue S1 and the second biological tissue S2 are sutured.

According to the needle carrier 100 of the first embodiment, since an operator causes the pinching arm member 21 to perform a pinching operation using the pinching arm opening/closing drive portion (the first drive part) 30, the pinching portion 22 can be closed to grasp the suturing needle 110. In addition, the pinching portion 22 can be pivoted around the second axis line O2 by the pinching arm pivotal drive portion (the second drive part) 40 to perform vertical handling of the needle (or change a posture of the object).

In addition, since the pinching arm opening/closing drive portion (the first drive part) 30 includes the axial dimension expansion/contraction spring 35 and the pair of pinching arm members 21 are biased toward a side at which the pinching portion 22 is closed by the axial dimension expansion/contraction spring 35 when the pinching operation is performed, occurrence of a play in the pinching portion 22 when the object such as a suturing needle or the like is pinched is minimized, and the object can be reliably pinched.

As a result, reliability when the suturing needle 110 is pinched can be improved while the operator can perform an easy and efficient operation.

In addition, since the pinching operation is transmitted to the pinching portion 22 via the axial dimension expansion/contraction spring 35, even when a large load is applied to the pinching operation input part, the load is attenuated and transmitted to the pinching portion 22 without being directly transmitted, and thus, occurrence of damage to the suturing needle 110 can be minimized.

In addition, since a large force transmitted to the pinching portion 22 via the pinching arm opening/closing drive portion (the first drive part) 30 is attenuated, occurrence of damage to the members that constitute the pinching arm opening/closing drive portion 30 is minimized, and the needle carrier 100 can be used for a long time while a decrease in reliability of the needle carrier 100 is minimized.

According to the needle carrier 100 of the first embodiment, since the pinching operation displacement absorption means is constituted by the axial dimension expansion/contraction spring 35 disposed on the flexible transmission member 34, a pinching operation transmission force can be efficiently converted into a biasing force at a side where the pinching arm member 21 is closed by a simple structure.

In addition, according to the needle carrier 100 of the first embodiment, since a suturing needle pinching concave portion 23 corresponding to the suturing needle 110 is formed in the pinching portion 22, the suturing needle 110 can be easily and stably grasped.

As a result, the suturing needle 110 can be stably grasped in a confined space, and handling of the needle can be efficiently and stably performed.

According to the pinching attachment 20 of the first embodiment, the pinching attachment 20 can be easily and efficiently attached to and detached from the needle carrier body 10.

As a result, the pinching attachment 20 is disposable, and hygienic safety in surgery can be improved.

### <Second embodiment>

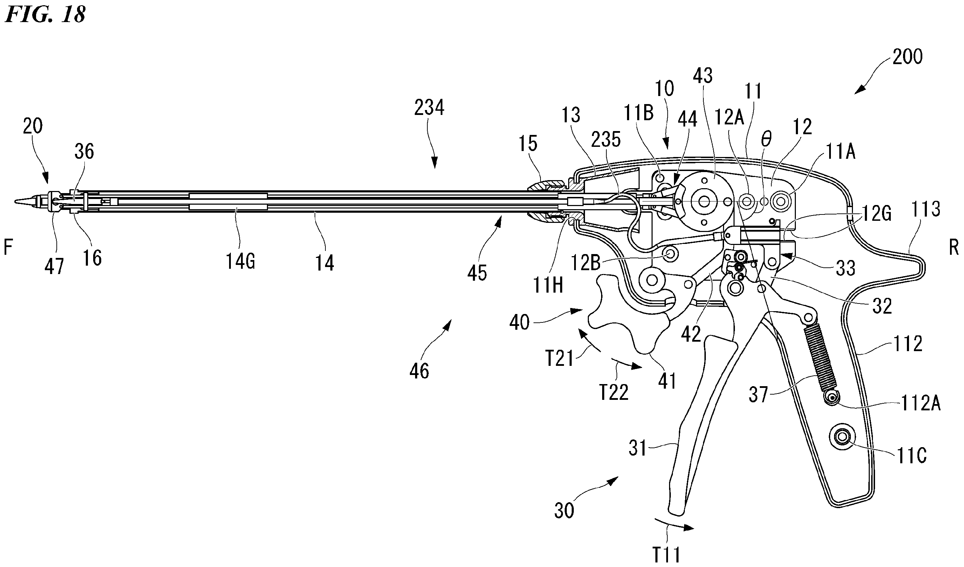

Hereinafter, a second embodiment of the present invention will be described with reference to Figs. 18 to 22.

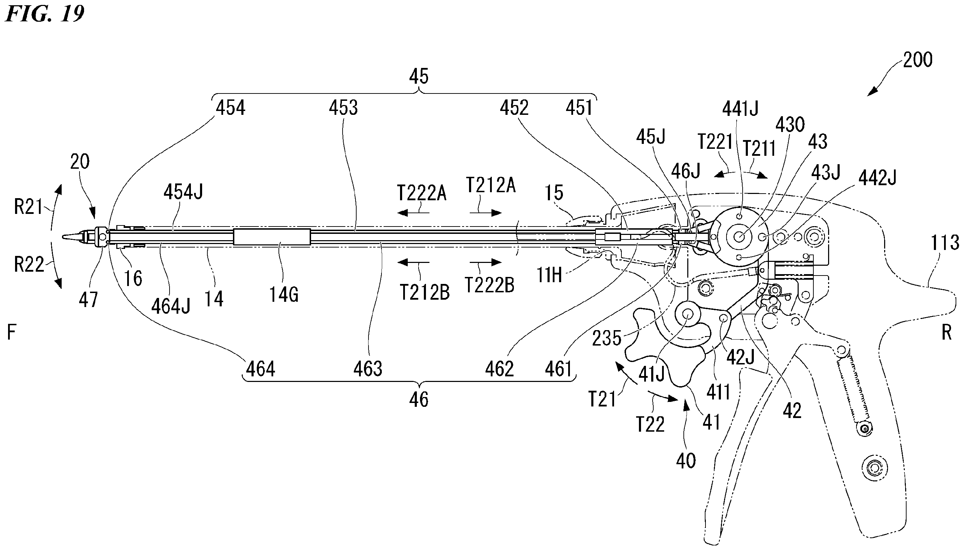

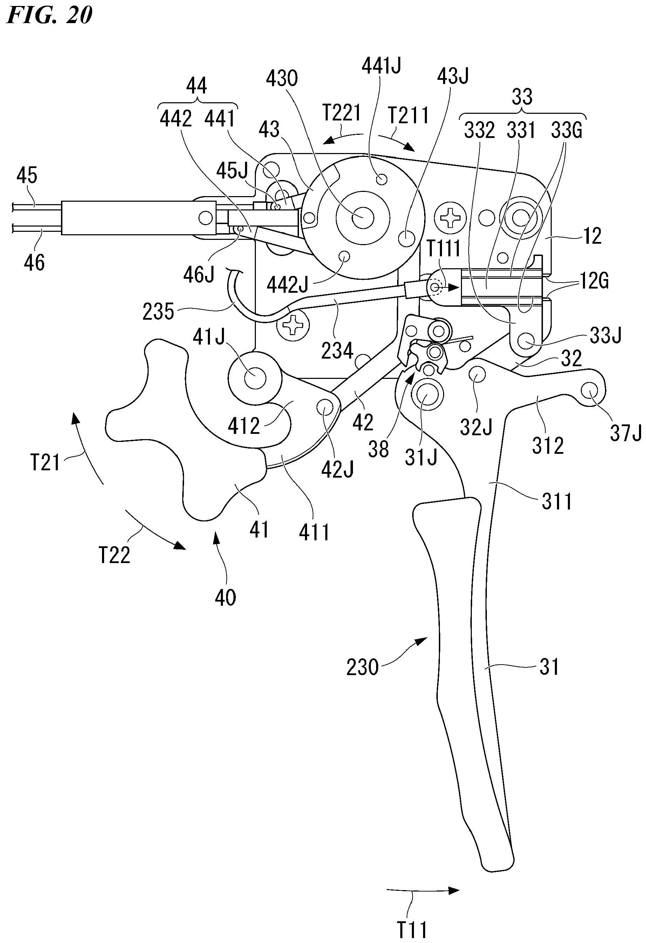

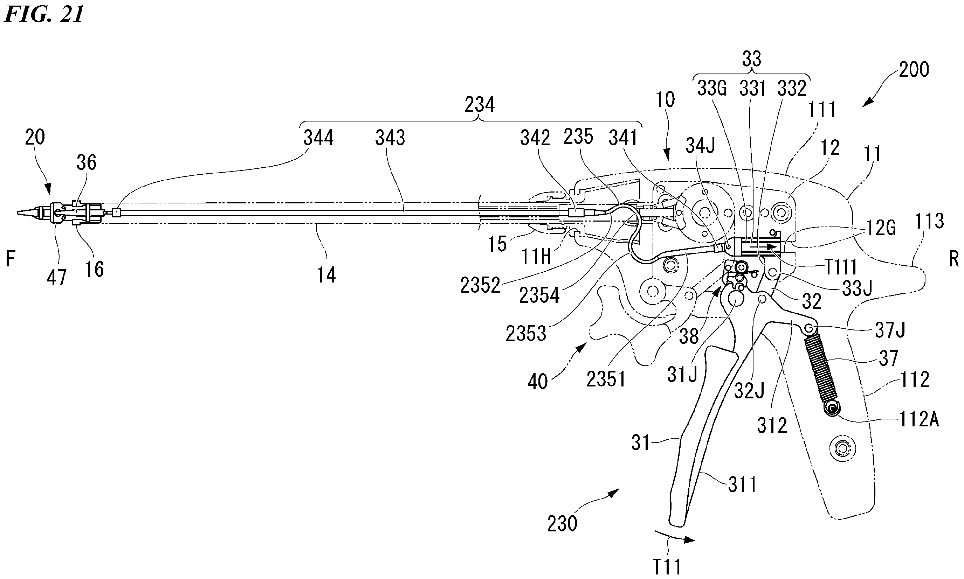

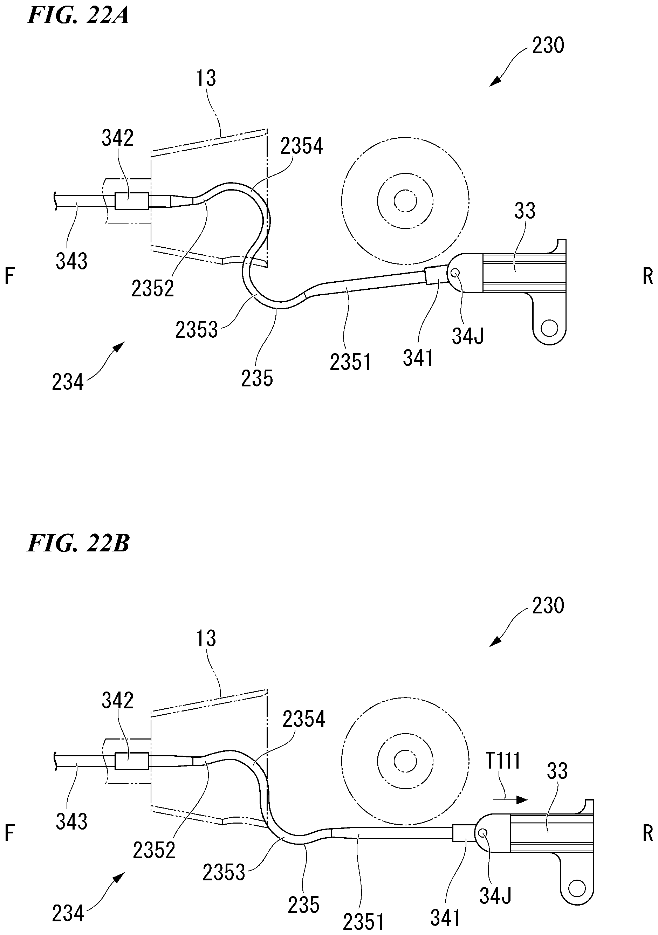

Fig. 18 is a longitudinal cross-sectional view explaining a schematic configuration of a needle carrier according to the second embodiment when seen in a side view, Fig. 19 is a longitudinal cross-sectional view explaining a schematic configuration of a pinching arm pivotal drive portion when seen in a side view, Fig. 20 is a longitudinal cross-sectional view explaining a schematic configuration of a pinching arm opening/closing drive portion when seen in a side view, Fig. 21 is a side view explaining major portions of the pinching arm opening/closing drive portion and the pinching arm pivotal drive portion in detail, Fig. 22A is a schematic configuration view showing an S-shaped spring in a state in which the pinching arm member is open, which is a view explaining an action of the pinching arm opening/closing drive portion, and Fig. 22B is a schematic configuration view showing the S-shaped spring in a state in which the pinching arm member is closed.

In the drawings, reference sign 200 designates a needle carrier (a grasper), reference sign 230 designates a pinching arm opening/closing drive portion (a first drive part), reference sign 234 designates a flexible transmission member, and reference sign 235 designates an S-shaped spring (an axial dimension expansion/contraction spring).

As shown in Figs. 18 and 19, the needle carrier (the grasper) 200 includes a needle carrier body (a grasper main body) 10, a pinching attachment (a grasping member) 20, the pinching arm opening/closing drive portion (the first drive part) 230 and a pinching arm pivotal drive portion (the second drive part) 40.

Then, the needle carrier (the grasper) 200 is configured such that the pinching arm member 21 is pivoted around the first axis line O1 to pinch the suturing needle (the object), and the pinching attachment 20 is pivoted around the second axis line O2 to enable handling of the suturing needle (the object) in a surface in a vertical tangential direction (a longitudinal direction of the needle carrier 200).

Since the needle carrier body (the grasper main body) 10, the pinching attachment (the grasping member) 20 and the pinching arm pivotal drive portion (the second drive part) 40 are the same as those of the first embodiment, they are designated by the same reference signs and description thereof will be omitted.

Further, the needle carrier 200 may be used as a grasper configured to grasp biological tissue (including a part), a piercing instrument, an incision instrument, or the like, as an object instead of the suturing needle.

Hereinafter, the pinching arm opening/closing drive portion (the first drive part) 230 will be described with reference to Figs. 20 to 22B.

Fig. 20 is a longitudinal cross-sectional view explaining a schematic configuration of the pinching arm opening/closing drive portion 230 according to the second embodiment when seen in a side view.

The pinching arm opening/closing drive portion (the first drive part) 230 has the pinching operation lever (the pinching operation part) 31 configured to cause the pair of pinching arm members 21 to perform a pinching operation, and a pinching operation transmission part configured to transmit the pinching operation from the pinching operation lever 31 to the pair of pinching arm members 21, and the pinching portion 22 is closed (pinched) by the pinching operation input to the pinching operation lever 31.

Specifically, as shown in Fig. 20, the pinching arm opening/closing drive portion (the first drive part) 230 includes the pinching operation lever (the pinching operation part) 31, the pinching operation transmission link 32, the guide member 33, the flexible transmission member 234, the pinching arm operating lever 36, the pinching operation return spring 37 and the pinching operation lock mechanism 38.

In addition, the pinching operation lever (the pinching operation part) 31, the pinching operation transmission link 32, the guide member 33, the flexible transmission member 234 and the pinching arm operating lever 36 are connected in this order.

In addition, as shown in Fig. 20, the flexible transmission member 234 includes the first transmission part 341, the second transmission part 342, the third transmission part 343, the fourth transmission part 344 and the S-shaped spring (the axial dimension expansion/contraction spring) 235, and the S-shaped spring 235 is disposed between the first transmission part 341 and the second transmission part 342.

Then, a longitudinal dimension of the flexible transmission member 234 (a widthwise dimension of the first transmission part 341 and the fourth transmission part 344) is expanded or contracted by the S-shaped spring 235.

Since configurations and materials of the first transmission part 341, the second transmission part 342, the third transmission part 343 and the fourth transmission part 344 are the same as those of the first embodiment, they are designated by the same reference signs and description thereof will be omitted.

For example, the S-shaped spring (the axial dimension expansion/contraction spring) 235 is constituted by a ribbon-shaped titanium alloy, and when seen in a side view, formed in an S shape having two curved portions that are curved in opposite directions.

In addition, as shown in Figs. 21, 21A and 21B, for example, the S-shaped spring (the axial dimension expansion/contraction spring) 235 includes a first end portion 2351 disposed on a first end side, a second end portion 2352 disposed on a second end side, a first curved portion 2353 disposed between the first end portion 2351 and the second end portion 2352, and a second curved portion 2354.

For example, the first end portion 2351 has a first end side extending toward the base end side R and connected to the first transmission part 341.

In addition, a second end side of the first end portion 2351 is connected to a first end side of the first curved portion 2353.

The second end portion 2352 is disposed at a position offset with respect to the first end portion 2351, for example, when seen along the axis line, and the first end side is connected to the second end (the tip side F) of the second curved portion 2354.

In addition, the second end portion 2352 extends toward a side opposite to the first end portion 2351 (the base end side R), and the second end side is connected to the second transmission part 342 disposed on the tip side F.

The first curved portion 2353 is formed in a curved shape protruding toward the tip side F, and the first end side is connected to the first end portion 2351.

The second curved portion 2354 is formed in a curved shape protruding toward the base end side R, and the second end side is connected to the second end portion 2352.

In addition, the second end side of the first curved portion 2353 (the end portion on the side opposite to the first end portion 2351) and the second end side of the second curved portion 2354 (the end portion on the side opposite to the second end portion 2352) are coaxially connected to each other.

In the embodiment, the first curved portion 2353 and the second curved portion 2354 are vertically arranged while protruding directions are opposite to each other and disposed in substantially an S shape.

The first curved portion 2353 and the second curved portion 2354 are offset, for example, when the first end portion 2351 and the second end portion 2352 are seen in the axial direction, and the first end portion 2351 and the second end portion 2352 extend in opposite directions.

The S-shaped spring (the axial dimension expansion/contraction spring) 235 is configured such that curvatures of the first curved portion 2353 and the second curved portion 2354 are decreased or increased when a load in an axial direction (a tensile direction, a compression direction) is applied between the first end portion 2351 and the second end portion 2352 according to the above-mentioned configuration.

In addition, the first curved portion 2353 and the second curved portion 2354 are not limited to the case in which they are curved when seen in a side view and may be disposed to be curved when seen in a plan view.

Next, an action of the pinching arm opening/closing drive portion 230 will be described with reference to Figs. 22A and 22B. Further, Fig. 22A is a schematic configuration view showing an S-shaped spring in a state in which the pinching arm member is open, and Fig. 22B is a schematic configuration view showing the S-shaped spring in a state in which the pinching arm member is closed.

### <Pinching operation>

Since the lock mechanism drive pin 381 in a state in which the pinching operation is not performed is the same as that of the first embodiment, description thereof will be omitted.
(1) In the S-shaped spring 235, in a state in which the pinching operation is not input, as shown in Fig. 22A, the curved portion is formed in an S shape having a large curvature (a small radius of curvature), the first transmission part 341 and the second transmission part 342 are in close proximity, and the pinching arm member 21 is in a state in which the pinching portion 22 is open.
(2) Next, when the pinching operation lever 31 for the pinching operation is pivoted in the arrow T11 direction and the guide member 33 is moved in the arrow Till direction to input the pinching operation via the first transmission part 341, as shown in Fig. 22B, a curvature of the curved portion of the S-shaped spring 235 is reduced, and the second transmission part 342 is biased to the base end side R.

Then, the biasing force is transmitted to the pinching arm operating lever 36 via the third transmission part 343 and the fourth transmission part 344, and the object such as the suturing needle or the like can be grasped in a state in which the left pinching arm member 21L is closed.

Here, in the S-shaped spring (the axial dimension expansion/contraction spring) 235, the curvatures of the first curved portion 2353 and the second curved portion 2354 are increased, as a result, even when an excessive load is applied between the first end portion 2351 and the second end portion 2352, displacement of the S-shaped spring 235 in the axial direction can be absorbed.

According to the needle carrier 200 of the second embodiment, when the operator causes the pinching arm member 21 to perform the pinching operation using the pinching arm opening/closing drive portion (the first drive part) 230, the pinching portion 22 can be closed to grasp the suturing needle. In addition, the pinching portion 22 can be pivoted around the second axis line O2 by the pinching arm pivotal drive portion (the second drive part) 40 to perform vertical handling of the needle (or change the posture of the object).

In addition, when the pinching arm opening/closing drive portion (the first drive part) 230 includes the S-shaped spring 235 and the pinching operation is performed, since the pair of pinching arm members 21 are biased toward a side where the pinching portion 22 is closed by the S-shaped spring 235, occurrence of a play in the pinching portion 22 when the object such as the suturing needle or the like is pinched can be minimized to reliably perform the pinching.

As a result, reliability when the suturing needle 110 is pinched can be improved while the operator can easily and efficiently perform the operation.

In addition, since the pinching operation is transmitted to the pinching portion 22 via the S-shaped spring 235, even when a large load is applied to the pinching operation input part, the load is attenuated and transmitted to the pinching portion 22 without being directly transmitted thereto, and thus, occurrence of damage to the suturing needle 110 can be minimized.

In addition, since the large force is attenuated and transmitted to the pinching portion 22 via the pinching arm opening/closing drive portion (the first drive part) 230, occurrence of damage to the members that constitute the pinching arm opening/closing drive portion 230 can be minimized, and the needle carrier 200 can be used for a long time while a decrease in reliability of the needle carrier 200 is minimized.

According to the needle carrier 200 of the second embodiment, since the pinching operation displacement absorption means is constituted by the S-shaped spring 235 disposed on the flexible transmission member 234, the pinching operation transmission force can be efficiently converted into a biasing force at the side where the pinching arm member 21 is closed by a simple structure.

### <Third embodiment

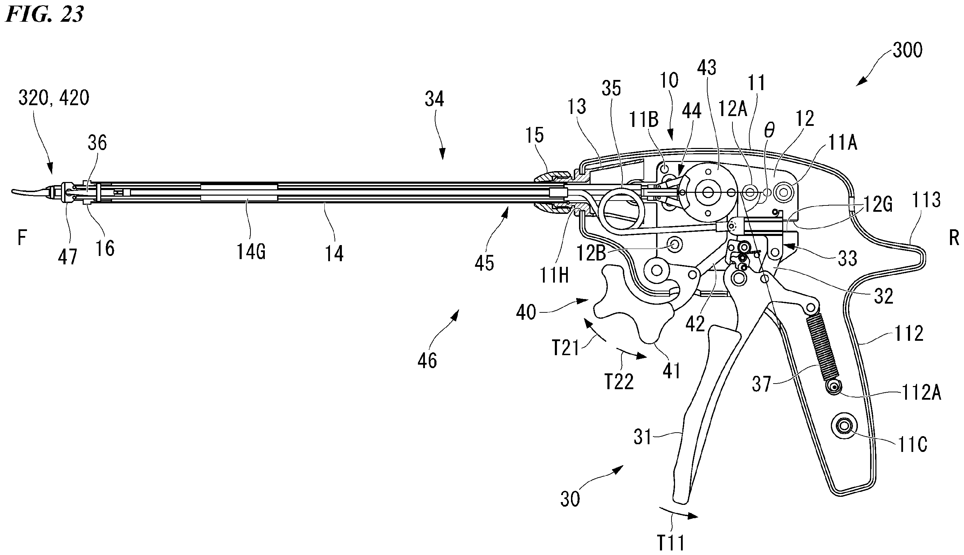

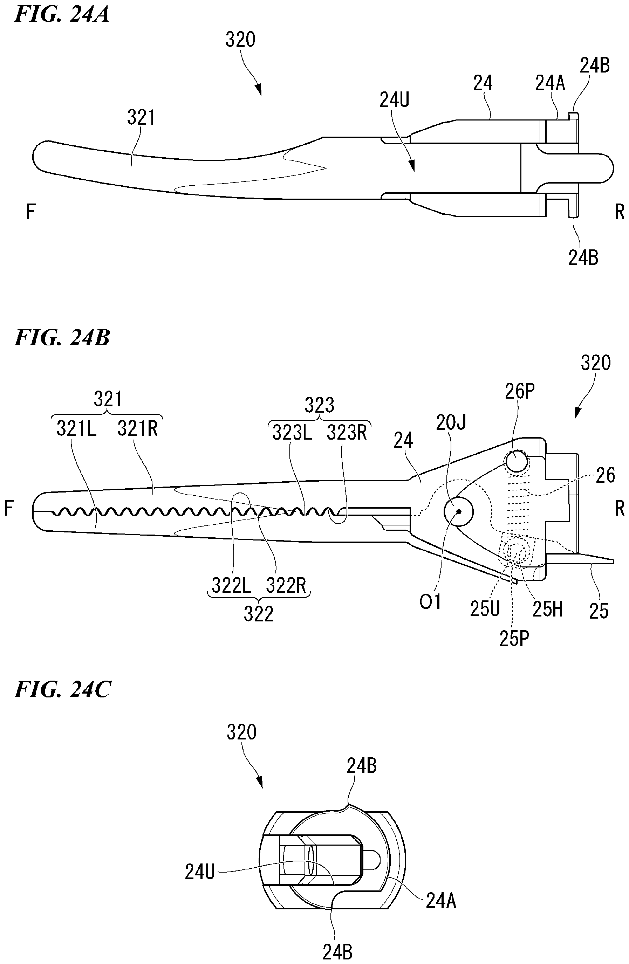

Next, an example of a grasper according to a third embodiment of the present invention will be described with reference to Figs. 23, and 24A to 24C.

Fig. 23 is a longitudinal cross-sectional view explaining a schematic configuration of the grasper according to the third embodiment of the present invention when seen in a side view. In addition, Figs. 23A to 23C are views explaining a schematic configuration of a forceps attachment, Fig. 23A is a schematic configuration view showing the forceps attachment when seen in a side view, Fig. 23B is a schematic configuration view when seen in a plan view, and Fig. 23C is a schematic configuration view when seen from a base end side.

In the drawings, reference sign 300 designates a grasper, and reference sign 320 designates a forceps attachment (a grasping member).

As shown in Fig. 23, for example, the grasper 300 includes a grasper main body 10, the forceps attachment (the pinching attachment, the grasping member) 320, the pinching arm opening/closing drive portion (the first drive part) 30 and the pinching arm pivotal drive portion (the second drive part) 40.

Since the grasper 300 according to the third embodiment is distinguished from the needle carrier 100 according to the first embodiment in that the forceps attachment 320 is provided instead of the pinching attachment (the grasping member) 20 and the others are the same as those of the first embodiment, description thereof will be omitted.

As shown in Figs. 24A to 24C, for example, the forceps attachment (the pinching attachment, the grasping member) 320 includes a pinching arm member 321, the pinching arm holding portion 24, the pinching operation receiving lever 25, the return spring (the pinching arm returning means) 26 and the support shaft 20J.

For example, the pinching arm member 321 includes a left pinching arm member 321L and a right pinching arm member 321R.

In addition, as shown in Fig. 24A, for example, the pinching arm member 321 is tapered from the base end side R toward the tip side F and has a curved portion that is slightly curved upward.

The entire length (a dimension in a longitudinal direction) of the pinching arm member 321 is, for example, 30 mm. In addition, a dimension from the tip of the left pinching arm member 321 to the first axis line O1 (a center of the support shaft 20J) is set to, for example, 22 mm.

In the left pinching arm member 321L, the pinching operation receiving lever 25 is connected to the base end side R, and a pivoting shaft receiving hole (not shown) is formed between the left pinching arm member 321L and the pinching operation receiving lever 25 in the longitudinal direction.

The right pinching arm member 321R constitutes a fixed pinching arm member, and the left pinching arm member 321L constitutes a movable pinching arm member.

Then, when the left pinching arm member 321L is pivoted around the first axis line O1, a pinching protrusion 322L and a pinching protrusion 322R approach or are separated from each other to pinch the object.

Then, the left pinching arm member 321L and the right pinching arm member 321R are relatively pivotable around the first axis line O1.

In addition, the pinching arm member 321 has a pinching protrusion 322 formed on the tip side F in the longitudinal direction.

The pinching protrusion 322 includes the pinching protrusion (the pinching portion) 322L and the pinching protrusion (the pinching portion) 322R. In addition, the pinching protrusion 322L and the pinching protrusion 322R are alternately differently formed in the longitudinal direction (from the base end side R to the tip side F).

Then, the pinching protrusion 322L and the pinching protrusion 322R approach each other to pinch the object, and the pinching protrusion 322L and the pinching protrusion 322R are separated from each other to release the object.

A V-shaped pinching concave portion 323L (323) is formed between the pinching protrusion (the pinching portion) 322L and the pinching protrusion (the pinching portion) 322L, which are adjacent to each other, for example, in a direction crossing (for example, perpendicular to) the longitudinal direction.

In addition, a V-shaped pinching concave portion 323R (323) is formed between the pinching protrusion (the pinching portion) 322R and the pinching protrusion (the pinching portion) 322R, which are adjacent to each other, for example, in a direction crossing (for example, perpendicular to) the longitudinal direction.

In the embodiment, the pinching concave portions 323L and 323R (323) are formed complementary to the pinching protrusions 322L and 322R (323).

Since the configurations of the pinching arm holding portion 24, the pinching operation receiving lever 25, the return spring (the pinching arm returning means) 26 and the support shaft 20J are the same as those in the first embodiment, they are designated by the same reference signs and description thereof will be omitted.

Further, for example, the forceps attachment 320 may be configured not to include the pinching protrusion 322 or the pinching concave portion 323.

In addition, the configuration and the dimension of the forceps attachment (the pinching attachment) 320 may be arbitrarily set.

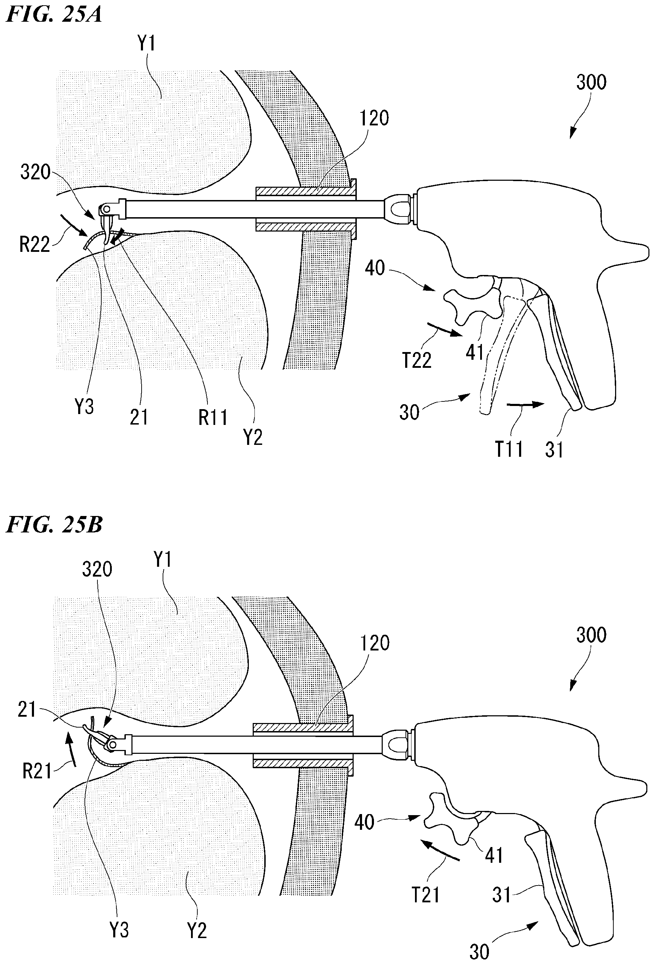

Next, an example of a method of using the grasper 300 will be described with reference to Figs. 25A and 25B.

Figs. 25A and 25B is a view explaining an example of an action of the grasper 300 to which the forceps attachment 320 according to the third embodiment is applied, for example, a conceptual view when a posture of a blood vessel Y3 disposed at a back side of biological tissues Y1 and Y2 is changed and treated in the abdominal cavity.

In Figs. 25A and 25B, reference sign 120 designates a trocar configured to insert the grasper 300 into the abdominal cavity in an abdominal cavity surgery.

(1) First, as shown in Fig. 25A, the tip side of the grasper 300 is inserted into the abdominal cavity via the trocar 120, and the forceps attachment 320 is disposed on the side behind the biological tissues (for example, an organ) Y1 and Y2. Here, it is preferable to move the pair of pinching arm members 321 in a closed state.
(2) Next, a pinching operation lock mechanism (not shown) is released, and the pinching arm member 321 becomes an open state.
   After that, as shown in Fig. 25A, the pivotal operation wheel 41 is operated in the arrow T22 direction, and the pinching arm member 321 is pivoted in the arrow R22 direction to dispose the pinching arm member 321 in the vicinity of the blood vessel Y3.
(3) Next, as shown in Fig. 25A, the pinching operation lever (the pinching operation part) 31 is operated in the arrow T11 direction, and the pinching arm member 21 is closed to grasp the blood vessel Y3.
   When the pinching arm member 321 is closed to grasp the blood vessel Y3, the pinching operation lock mechanism (not shown) is operated, and a closed state of the pinching arm member 321 is held.
(4) Next, as shown in Fig. 25B, the pivotal operation wheel (the pivotal operation part) 41 is operated in the arrow T21 direction, and the pinching arm member 321 is pivoted in the arrow R21 direction to change an orientation of the blood vessel Y3 while the blood vessel Y3 is pinched. After that, desired treatment will be continuously performed.

According to the grasper 300 of the third embodiment, since the pinching arm member 321 of the forceps attachment 320 is curved toward the tip side, for example, a part of a living body such as the blood vessel Y3 or the like, or a grasping object of biological tissue can be easily grasped.

In addition, according to the grasper 300, a position or a posture of the grasping object of the blood vessel Y3 can be easily and efficiently changed in a confined space.

In addition, according to the grasper 300, for example, it is possible to prevent a grasping force when the biological tissue such as the blood vessel Y3 or the like is grasped from becoming too large, and it is possible to prevent the blood vessel Y3 or the like from being damaged.

### <Fourth embodiment>

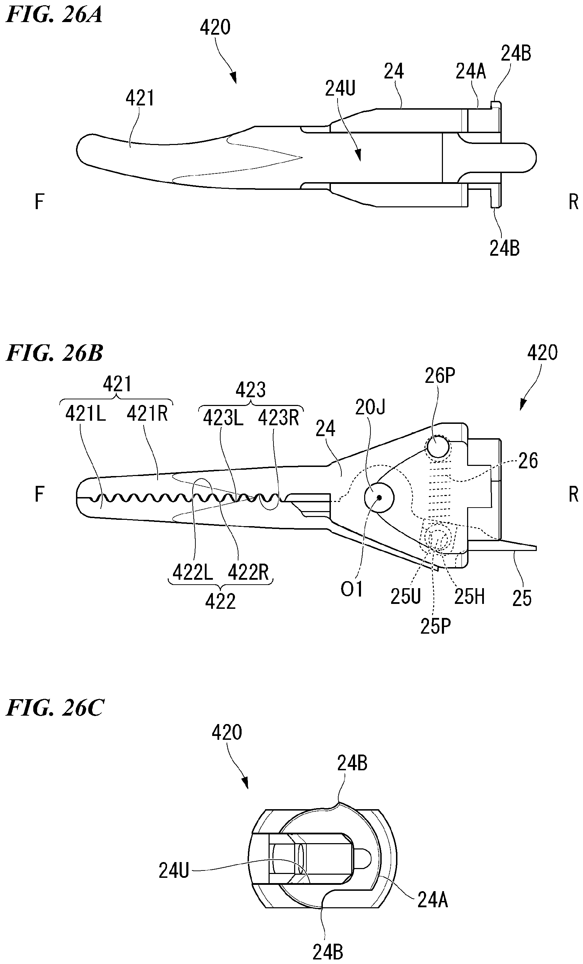

Next, an example of a forceps attachment according to a fourth embodiment of the present invention will be described with reference to Figs. 26A to 26C.

Figs. 26A to 26C are views explaining a schematic configuration of the forceps attachment according to the fourth embodiment of the present invention, Fig. 26A is a schematic configuration view of the forceps attachment when seen in a side view, Fig. 26B is a schematic configuration view when seen in a plan view, and Fig. 26C is a schematic configuration view when seen from a base end side.

In the drawings, reference sign 420 shows a forceps attachment (a grasping member).

As shown in Figs. 26A to 26C, for example, the forceps attachment (the pinching attachment, the grasping member) 420 includes a pinching arm member 421, a pinching arm holding portion 24, a pinching operation receiving lever 25, a return spring (a pinching arm returning means) 26 and a support shaft 20J.

The pinching arm member 421 includes, for example, a left pinching arm member 421L and a right pinching arm member 421R.

In addition, as shown in Fig. 24A, for example, the pinching arm member 421 is tapered from the base end side R toward the tip side F, and becomes a curved portion having a larger curvature than that of the pinching arm member 321 according to the third embodiment.

The entire length (a dimension in a longitudinal direction) of the pinching arm member 421 is formed as, for example, 23.4 mm. In addition, a dimension from the tip of the left pinching arm member 421 to the first axis line O1 (the center of the support shaft 20J) is set to, for example, 15.4 mm.

A pinching protrusion 422 (422L, 422R) and a pinching concave portion 423 (423L, 423R) are formed on the pinching arm member 421 (421L, 421R).

Since the pinching protrusion 422 (422L, 422R) and the pinching concave portion 423 (423L, 423R) are the same as the pinching protrusion 322 (322L, 322R) and the pinching concave portion 323 (323L, 323R) according to the third embodiment, description thereof will be omitted.

In addition, since the configurations of the pinching arm holding portion 24, the pinching operation receiving lever 25, the return spring (the pinching arm returning means) 26 and the support shaft 20J are the same as those of the first embodiment, they are designated by the same reference signs, and description thereof will be omitted.

Further, the configuration and the dimension of the forceps attachment (the pinching attachment) 420 can be arbitrarily set.

Further, the present invention is not limited to the above-mentioned embodiments and various modifications may be made without departing from the spirit of the present invention.

For example, while the case in which the pinching arm opening/closing drive portion (the first drive part) 30 or 230 includes the pinching operation lever 31, the pinching operation transmission link 32, the guide member 33, the flexible transmission member 34 or 234, the pinching arm operating lever 36, the pinching operation return spring 37 and the pinching operation lock mechanism 38 has been described in the above-mentioned embodiments, the configuration of the pinching arm opening/closing drive portion 30 or 230 may be arbitrarily set, and a configuration in which the pinching attachment (the pinching member) 20 or the forceps attachment (the pinching member) 320 (420) is pinched by a first drive part having another configuration may be provided.

In addition, the configuration of the pinching operation part may be arbitrarily set, and the pinching operation part may be constituted by a lever or the like instead of the pivotal operation wheel.

In addition, while the case in which the flexible transmission member 34 or 234 is formed of a titanium alloy has been described in the embodiments, the configuration and the material of the flexible transmission member 34 or 234 may be appropriately set, and for example, the member may be formed of a material other than the titanium alloy.

In addition, while the case in which the pinching operation displacement absorption means is disposed on the flexible transmission member 34 or 234 has been described in the above-mentioned embodiments, the configuration and the disposed position of the pinching operation displacement absorption means may be arbitrarily set, and for example, it may be disposed separately from the flexible transmission member 34 or 235.

In addition, while the case in which the pinching operation displacement absorption means is constituted by the axial dimension expansion/contraction spring 35 including the winding portion (the circumferential portion) 353 and the S-shaped spring 235 has been described in the above-mentioned embodiments, another type of an axial dimension expansion/contraction spring constituted by an air spring or a spiral spring may be applied thereto.

In addition, while the case in which the needle carrier 100 or 200 and the grasper 300 includes the pinching operation lock mechanism 38 and the pinching state of the pinching arm member 21 may be locked and released by the pinching operation lock mechanism 38 has been described in the above-mentioned embodiments, whether the pinching arm lock mechanism38 is provided may be arbitrarily set, and the pinching operation lock mechanism 38 may be configured to only lock the pinching state. In addition, the configuration of the pinching operation lock mechanism may be appropriately set.

In addition, while the case in which the pinching operation lock mechanism 38 has the ratchets 383S and 383T configured to stop the pinching operation at one step has been described, the pinching arm member 21 may be configured to be stopped at a plurality of positions.

In addition, while the case in which the pinching arm pivotal drive portion (the second drive part) 40 includes the pivotal operation wheel (the pinching operation input part) 41, the pivotal operation transmission link 42, the pivoting control cam 43, the transmission link 44, the first transmission member 45, the second transmission member 46 and the grasping member pivoting portion 47 has been described in the above-mentioned embodiments, for example, a configuration in which a pivotal operation is transmitted from the pivotal operation wheel (the pinching operation input part) 41 to the grasping member pivoting portion 47 may be arbitrarily set, and a configuration in which the grasping member pivoting portion is directly pivoted by the pivotal operation part without including the pivotal operation transmission part may be provided.

In addition, the configurations of the pivotal operation part and the grasping member pivoting portion may be arbitrarily set.

In addition, while the case in which the needle carrier 100 or 200 and the grasper 300 includes the pinching arm opening/closing drive portion (the first drive part) 30 or 230 and the pinching arm pivotal drive portion (the second drive part) 40 has been described in the above-mentioned embodiments, for example, a configuration in which only the pinching arm opening/closing drive portion (the first drive part) is provided and the grasping member (the pinching attachment, the forceps attachment, or the like) is pinched by the pinching arm opening/closing drive portion without a pivotal operation may be provided.

In addition, while the case in which the left pinching arm member 21L, 321L or 421L of the pair of pinching arm members 21, 321 or 421 is pivoted around the first axis line O1 has been described in the above-mentioned embodiments, a configuration in which the left pinching arm member 21L, 321L or 421L and the right pinching arm member 21R, 321R or 421R are pivoted around the first axis line O1 may be provided.

In addition, while the case in which the pinching attachment 20, 320 or 420 is configured such that the pinching member is detachable from the pinching arm opening/closing drive portion (the first drive part) 30 or 230 and the pinching arm pivotal drive portion (the second drive part) 40 has been described in the above-mentioned embodiments, a configuration in which the pinching member is fixed to the pinching arm opening/closing drive portion 30 or 230 or the pinching arm pivotal drive portion 40 may be provided.

In addition, while the case in which the pinching attachment 20 or the forceps attachment 320 or 420 includes the pair of pinching arm members 21, 321 or 421 and the pinching arm holding portion 24 and the pinching attachment 20 is mounted on the grasping member pivoting portion 47 has been described in the above-mentioned embodiment, the configuration of the pinching attachment may be arbitrarily set. In addition, for example, the pinching attachment 20 or the forceps attachment 320 or 420 may include the grasping member pivoting portion 47 or may have an arbitrary attachment relationship.

### [Industrial Applicability]

According to a grasper, a needle carrier and a pinching attachment of the present invention, since they can be easily and efficiently operated by an operator, reliability of a pinching operation when the object is pinched can be improved, and the operation can be easily and efficiently performed, they are industrially applicable.

### [Reference Signs List]

O1 First axis line
O2 Second axis line
F Tip side
R Base end side
10 Needle carrier body (grasper main body)
11 Casing
11H Attachment hole
111 Casing main body
112 Grip portion
12 Base plate
14 Protection pipe
20 Pinching attachment (grasping member)
21 Pinching arm member (pair of pinching arm member)
21L Left pinching arm member
21R Right pinching arm member
22 Pinching portion
22L, 22R Pinching protrusion (pinching portion)
23 Suturing needle pinching concave portion
24 Pinching arm holding portion
24B Flange portion (second engaging portion)
25 Pinching operation receiving lever
26 Return spring (pinching arm returning means)
30, 230 Pinching arm opening/closing drive portion (first drive part)
31 Pinching operation lever (pinching operation part)
32 Pinching operation transmission link
33 Guide member
34, 234 Flexible transmission member
35 Axial dimension expansion/contraction spring (pinching operation displacement absorption means)
353 Winding portion (circumferential portion)
36 Pinching arm operating lever
37 Pinching operation lever return spring
38 Pinching operation lock mechanism (pinching operation lock mechanism, pinching operation release mechanism)
40 Pinching arm pivotal drive portion (second drive part)
41 Pivotal operation wheel (pivotal operation part)
47 Grasp member pivoting portion
47B Flange portion (first engaging portion)
100, 200 Needle carrier (grasper)
110 Suturing needle (object)
235 S-shaped spring (pinching operation displacement absorption means)
2353 First curved portion (curved portion)
2354 Second curved portion (curved portion)
300 Grasper
320, 420 Pinching attachment (grasping member)
321, 421 Pinching arm member (pair of pinching arm member)
321L, 421L Left pinching arm member
321R, 321L Right pinching arm member
322, 322L, 322R, 422R, 422L, 422R Pinching protrusion (pinching portion)
323 Object pinching concave portion

## Claims

1. A grasper configured to grasp an object, comprising:
a grasping member including a pair of pinching arm members having pinching portions formed on a tip side in a longitudinal direction and configured to pinch an object, which relatively pivot around a first axis line to cause the pinching portions to approach each other or be separated from each other, and a pinching arm holding portion on which the pair of pinching arm members are pivotably disposed around the first axis line;
a first drive part including a pinching operation part configured to pinch the pair of pinching arm members and a pinching operation transmission part configured to transmit a pinching operation from the pinching operation part to the pair of pinching arm members, in which the first drive part is configured to cause the pinching portions to approach each other according to the pinching operation input to the pinching operation part;
a second drive part including a pivotal operation part configured to pivot the pair of pinching arm members, and a grasping member pivoting portion configured to pivot the grasping member around a longitudinal direction of the pinching arm member and around a second axis line perpendicular to the first axis line according to the pivotal operation input from the pivotal operation part; and
an elongated grasper main body having a grip portion held by an operator in an orientation crossing the longitudinal direction, in which the grasping member, the first drive part and the second drive part are disposed on the elongated grasper main body,
wherein the first drive part comprises a pinching operation displacement absorption means configured to bias the pair of arm members to cause the pinching portions to relatively approach each other according to the pinching operation input from the pinching operation part.

2. The grasper according to claim 1, wherein the pinching operation displacement absorption means is constituted by a flexible transmission member disposed on the pinching operation transmission part and operated by a pinching operation transmission force.

3. The grasper according to claim 2, wherein the pinching operation displacement absorption means is constituted by an axial dimension expansion/contraction spring.

4. The grasper according to claim 2, wherein the pinching operation displacement absorption means is constituted by an axial dimension expansion/contraction spring having a circumferential portion surrounded by an elastic material.

5. The grasper according to claim 2, wherein the pinching operation displacement absorption means is constituted by an S-shaped axial dimension expansion/contraction spring constituted by curved portions on which elastic materials are directed toward opposite sides.

6. The grasper according to any one of claims 1 to 5, comprising a pinching operation lock mechanism configured to automatically hold a pinching state of the pinching portions by operating the pinching operation part.

7. The grasper according to claim 6, comprising a pinching operation release mechanism configured to release the pinching state of the pinching portions using the pinching operation part by operating the pinching operation part in a state in which the pinching operation lock mechanism holds the pinching state of the pinching portions.

8. A needle carrier comprising the grasper according to any one of claims 1 to 7,
wherein a suturing needle pinching concave portion corresponding to a suturing needle is formed in the pinching portions.

9. A pinching attachment comprising a pinching arm operating lever constituting the first drive part according to any one of claims 1 to 8 and configured to transmit a pinching operation to the pair of pinching arm members, and a grasping member pivoting portion constituting the second drive part and configured to pivot the grasping member around the second axis line, and used by being mounted on a grasper having a first engaging portion configured to detachably mount the grasping member on the grasping member pivoting portion,
wherein one of the pair of pinching arm members is connected to a tip side of the pinching arm holding portion and a pinching operation receiving lever configured to receive a pinching operation from the pinching arm operating lever is connected to a base end side of the other of the pair of pinching arm members,
the pinching operation receiving lever is disposed at a position corresponding to the pinching arm operating lever via a through-hole formed from the tip side toward the base end side of the pinching arm holding portion,
a second engaging portion corresponding to the first engaging portion is formed in the pinching arm holding portion on the base end side, the first engaging portion and the second engaging portion have flange portions configured to set an attachment position in the attachment/detachment direction in a state in which a boss formed on any one thereof is inserted into a hole formed in the other and to prevent the boss from falling out in the attachment/detachment direction, and the flange portions are engaged with each other by being pivoted around an axis line in the attachment/detachment direction at the attachment position and causing the flange portions to overlap each other, and
the pinching operation receiving lever is configured to be insertable between the pinching arm operating lever and the pinching arm holding portion by causing the pinching portions of the pair of pinching arm members to approach each other in a state in which the first engaging portion and the second engaging portion are twisted to a position in which they are movable in the attachment/detachment direction.
